(19) **Europäisches Patentamt / European Patent Office / Office européen des brevets**

(11) **EP 3 597 731 B1**

(12) **EUROPEAN PATENT SPECIFICATION**

(45) Date of publication and mention
of the grant of the patent:
**29.06.2022 Bulletin 2022/26**

(51) International Patent Classification (IPC):
**C12N 5/071** (2010.01)    **C07K 14/78** (2006.01)
**C12N 1/00** (2006.01)    **C12N 15/09** (2006.01)
**C12N 5/00** (2006.01)    **C12N 5/0775** (2010.01)

(21) Application number: **18767668.9**

(22) Date of filing: **16.03.2018**

(52) Cooperative Patent Classification (CPC):
**C07K 14/78; C12N 1/00; C12N 5/0062;**
**C12N 5/0662;** C12N 15/09; C12N 2533/54;
C12N 2535/10

(86) International application number:
**PCT/JP2018/010535**

(87) International publication number:
**WO 2018/169065 (20.09.2018 Gazette 2018/38)**

(54) **METHOD FOR PRODUCING CELL STRUCTURES**

VERFAHREN ZUR HERSTELLUNG VON ZELLSTRUKTUREN

PROCÉDÉ DE PRODUCTION DE STRUCTURES CELLULAIRES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **17.03.2017 JP 2017052953**

(43) Date of publication of application:
**22.01.2020 Bulletin 2020/04**

(73) Proprietors:
• **FUJIFILM Corporation**
**Tokyo 106-8620 (JP)**
• **KYOTO PREFECTURAL PUBLIC UNIVERSITY
CORPORATION**
**Kyoto 602-8566 (JP)**

(72) Inventors:
• **NAKAMURA, Kentaro**
**Ashigarakami-gun**
**Kanagawa 258-8577 (JP)**

• **HANDO, Rie**
**Ashigarakami-gun**
**Kanagawa 258-8577 (JP)**
• **GOJO, Satoshi**
**Kyoto-shi**
**Kyoto 602-8566 (JP)**
• **KAMI, Daisuke**
**Kyoto-shi**
**Kyoto 602-8566 (JP)**

(74) Representative: **Hoffmann Eitle**
**Patent- und Rechtsanwälte PartmbB**
**Arabellastraße 30**
**81925 München (DE)**

(56) References cited:
EP-A1- 3 156 081    EP-A1- 3 358 003
EP-A1- 3 473 259    WO-A1-2012/036011
WO-A1-2014/133081    WO-A1-2019/004446

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**BACKGROUND OF THE INVENTION**

1. Field of the Invention

The present invention relates to a method for manufacturing a cell structure which includes a biocompatible macromolecular block and a cell and in which the plurality of biocompatible macromolecular blocks are disposed in gaps between the plurality of cells.

2. Description of the Related Art

Currently, regenerative medicine, which regenerates living body tissues and organs having functional disorders or dysfunction, is put into practical use. The regenerative medicine is new medical technology creating a form or a function of a living body tissue that cannot be recovered with only natural healing ability possessed by a living body, which is the same as that of an original tissue, again, using three factors including a cell, a scaffold, and a growth factor. In recent years, treatment using cells is gradually realized. Examples thereof include cultured epidermis using autologous cells, a cartilage treatment using autologous chondrocytes, a bone regeneration treatment using mesenchymal stem cells, a cardiomyocyte sheet treatment using myoblasts, a cornea regeneration treatment using a corneal epithelial sheet, and a nerve regeneration treatment. These new treatments are intended to repair and regenerate a living tissue, unlike alternative medicine by an artificial object (for example, artificial bone substitutes and hyaluronic acid injection) in the related art, and can achieve high therapeutic effects. In practice, products such as cultured epidermis and cultured cartilage using autologous cells have been commercially available.

In Patent Document 1, a cell structure that includes a macromolecular block having biocompatibility and a cell and in which the plurality of macromolecular blocks are disposed in gaps between the plurality of cells is disclosed. In the cell structure disclosed in Patent Document 1, nutrient from the outside to the inside of the cell structure can be delivered, the cell has a sufficient thickness, and cells are uniformly present in the structure. In the example of Document 1, high cell survival activity is demonstrated by using a macromolecular block formed of recombinant gelatin or a natural gelatin material.

In Document 2, a cell structure for cell transplantation that includes a macromolecular block having biocompatibility and at least one type of cells and in which the plurality of macromolecular blocks are disposed in gaps between the plurality of cells is disclosed.

In Patent Document 3, a cell structure for cell transplantation which includes biocompatible macromolecular blocks not including glutaraldehyde and at least one kind of cells and in which the plurality of biocompatible macromolecular blocks are disposed in gaps between the plurality of cells, wherein a tap density of the biocompatible macromolecular blocks is 10 mg/cm$^3$ to 500 mg/cm$^3$, or a value obtained by dividing a square root of a cross sectional product in a two-dimensional cross sectional image of the macromolecular blocks by a perimeter is 0.01 to 0.13 is disclosed.

In Patent Documents 1 to 3 as mentioned above, manufacturing methods in a small scale of manufacturing one cell structure that includes macromolecular blocks having biocompatibility and cells and in which the plurality of macromolecular blocks are disposed in gaps between the plurality of cells per one well are disclosed.

On the other hand, Patent Document 4 discloses a culture substrate in which a plurality of recessed portions forming compartments in which an object to be cultured is cultured are formed on the surface of the culture substrate, and the surface of the culture substrate between the recessed portions adjacent to each other is a non-flat surface, and discloses that spheroid culture is carried out using this culture substrate.

Patent Document 5 discloses a cell culture container for performing spheroid culture which includes: a culture surface on which a plurality of recessed portions forming compartments in which an object to be cultured is cultured are formed; a container main body including the culture surface on a bottom surface, and a liquid-permeable lid body which is placed on the top portions of the plurality of recessed portions and closes the openings of the recessed portions, in which the top portion of the culture surface between the recessed portions adjacent to each other, the liquid-permeable lid body is disposed so that the distance from the top portion between the recessed portions in a state of being immersed in a culture liquid in the container main body is smaller than the outer diameter dimension of the object to be cultured which has been cultured in the recessed portions.

EP 3 156 081 discloses a cell structure for use in treatment of brain injury, a method for producing the same and a therapeutic agent for brain injury. EP 3 358 003 discloses a method for manufacturing a sheet-shaped cell structure, and a sheet-shaped cell structure. EP 3 473 259 discloses a trophic factor release agent and an inflammatory disease treatment agent.

Prior Art Document

Patent Documents

Patent Document 1: WO2011/108517A
Patent Document 2: JP2014-012114A
Patent Document 3: WO2014/133081A
Patent Document 4: WO2012/036011A
Patent Document 5: JP2015-073520A

## SUMMARY OF THE INVENTION

In a case of being used in repair and regeneration of a living tissue, for example, it is desirable to simultaneously manufacture cell structures in large quantities, for example, 1,000 or more cell structures in some cases. As a method of culturing a large amount of cell mass, a method of suspending and stirring the cell mass in a medium has been known. However, the inventors of the present invention have been found that, in a case where a suspending, stirring, and culturing method is used for manufacturing of a cell structure, there are problems in that, (1) the formation efficiency of the cell structure is poor (that is, a cell structure is not formed, and a ratio of the macromolecular blocks and the cells which are individually present is large), (2) a ratio of the macromolecular blocks and the cells in the obtained cell structure varies for each cell structure, and (3) sizes of the cell individual structures are different from each other. In a case where the cell structure is used for repair and regeneration of a living tissue, it is desirable that the cell structure is homogeneous (for example, a ratio, a shape, or a size of macromolecular blocks and cells is homogeneous) in order to obtain an excellent therapeutic effect.

An object of the present invention is to provide a method of manufacturing a large amount of uniform cell structures that include biocompatible macromolecular blocks and cells and in which the plurality of biocompatible macromolecular blocks are disposed in gaps of the plurality of cells for a short period of time.

The inventors of the present invention have diligently researched to achieve the above object to find that it is possible to manufacture a large amount of uniform cell structures for a short period of time by adding a mixture of biocompatible macromolecular blocks, cells, and a liquid medium to a first culture container having a culture surface on which the plurality of recessed portions are formed and a side wall part standing on an outer periphery of the culture surface and allowing the first culture container to stand, so as to form cell structures, and then stirring and culturing the cell structures in a second culture container comprising stirring means. The present invention has been completed based on the finding.

That is, the present invention provides.

(1) A method of manufacturing a cell structure, comprising: a step (A) of adding a mixture of biocompatible macromolecular blocks, cells, and a liquid medium to a first culture container having a culture surface on which a plurality of recessed portions are formed and a side wall part standing on an outer periphery of the culture surface such that a liquid surface of the mixture is over the culture surface;

a step (B) of allowing a first culture container of the step (A) to stand and forming a cell structure which includes the biocompatible macromolecular block and the cell in the recessed portion and in which the plurality of biocompatible macromolecular blocks are disposed in gaps between the plurality of cells; and
a step (C) of stirring and culturing a content of the first culture container obtained in the step (B) in a second culture container comprising stirring means, (i) wherein first culture container is allowed to stand in the step (B) for a period of time for which a ratio of the number of cell structures manufactured after the step (C) to the number of recessed portions is 70% or more , (ii) wherein the recessed portions are formed by 10 recessed portions/cm^2 to 10,000 recessed portions/cm^2 per unit area of the well forming area (24), and (iii) wherein the cells to be used are one or more selected from the group consisting of ES cells, iPS cells, MSCs, chondrocytes, osteoblasts, osteoprecursor cells, mesenchymal cells, myoblasts, cardiac muscle cells, cardiomyoblasts, nerve cells, hepatocytes, beta cells, fibroblasts, corneal endothelial cells, vascular endothelial cells, corneal epithelial cells, amniotic cells, umbilical cord blood cells, bone marrow derived cells, and hematopoietic stem cells.

(2) The method of manufacturing a cell structure according to (1), in which the first culture container is allowed to stand in the step (B) until free biocompatible macromolecular blocks are 30 mass% or less of the total biocompatible macromolecular blocks.

(4) The method of manufacturing a cell structure according to any one of (1) to (2), in which a size of the biocompatible macromolecular block is 1 $\mu$m to 700 $\mu$m.

(5) The method of manufacturing a cell structure according to any one of (1) to (4), in which a treatment for suppressing cell adhesion is performed on the culture surface and a surface of the recessed portion.

(6) The method of manufacturing a cell structure according to any one of (1) to (5), in which a depth of the recessed portion is 2 to 100 times of a size of the biocompatible macromolecular block, and a diameter of the recessed portion is 2 to 100 times of a size of the biocompatible macromolecular block.

(7) The method of manufacturing a cell structure according to any one of (1) to (6), in which a period of time for which the first culture container is allowed to stand is 2 hours to 24 hours.

(8) The method of manufacturing a cell structure according to any one of (1) to (7), in which stirring and culturing time for which a content of the first culture container in the step (C) is 12 hours to 93 hours.

(9) The method of manufacturing a cell structure according to any one of (1) to (8), in which the biocompatible macromolecule is gelatin.

(10) The method of manufacturing a cell structure according to (9), in which the gelatin is represented by the following formula.

$$\text{Formula: } A-[(Gly-X-Y)_n]_m-B$$

in the formula, A represents random amino acid or an amino acid sequence, B represents any amino acid or amino acid sequence, n X's each independently represent any one of amino acid, n Y's each independently represent any one of any amino acid, n represents an integer of 3 to 100, and m represents an integer of 2 to 10, and also n Gly-X-Y's may be identical to or different from each other.

(11) The method of manufacturing a cell structure according to (9) or (10), in which the gelatin is any one of

a peptide including an amino acid sequence described in SEQ ID NO: 1;

a peptide having biocompatibility and including an amino acid sequence in which one or more amino acids are deleted, substituted, or added in an amino acid sequence described in SEQ ID NO: 1; or

a peptide which is formed of an amino acid sequence having 80% or more sequence identity to the amino acid sequence described in SEQ ID No: 1, and has biocompatibility.

According to a method of manufacturing a cell structure of the present invention, it is possible to manufacture a large amount of uniform cell structures that include biocompatible macromolecular blocks and cells and in which the plurality of biocompatible macromolecular blocks are disposed in gaps of the plurality of cells for a short period of time.

## BRIEF DESCRIPTION OF THE DRAWINGS

Fig. 1 is a perspective view of a first culture container.

Figs. 2A and 2B are cross-sectional views of a first example of the first culture container (an upper surface of an area between recessed portions, that is, the most upper portion of the recessed portion is non-flat). Fig. 2A illustrates an example without a cell adhesion suppressant layer and Fig. 2B shows an example with the cell adhesion suppressant layer.

Figs. 3A and 3B are cross-sectional views of a second example of a first culture container (an upper surface of an area between recessed portions, that is, the most upper portion of the recessed portion is flat). Fig. 3A illustrates an example without a cell adhesion suppressant layer and Fig. 3B illustrates an example with the cell adhesion suppressant layer.

Fig. 4 is a partially enlarged view of Fig. 2A.

Fig. 5 is a partially enlarged view of Fig. 3A.

Fig. 6 illustrates spots irradiated with laser light.

Fig. 7 illustrates a liquid temperature profile of a condition A of examples.

Fig. 8 illustrates a liquid temperature profile of a condition B of examples.

Fig. 9 illustrates a liquid temperature profile of a condition C of examples.

Fig. 10 illustrates a manufacturing step and results of Example 1.

Fig. 11 illustrates a manufacturing step and results of Example 2.

Fig. 12 illustrates a manufacturing step and results of Comparative Example 1.

Fig. 13 illustrates a manufacturing step and results of Comparative Example 2.

Fig. 14 illustrates a manufacturing step and results of Comparative Example 3.

## DESCRIPTION OF THE PREFERRED EMBODIMENTS

Hereinafter, an embodiment of the present invention will be described in detail.

A method of manufacturing a cell structure according to the embodiment of the present invention includes

a step (A) of adding a mixture of biocompatible macromolecular blocks, cells, and a liquid medium, to a first culture container having a culture surface, on which a plurality of recessed portions are formed, and a side wall part standing on an outer periphery of the culture surface such that a liquid surface of the mixture is over the culture surface;

a step (B) of allowing a first culture container of the step (A) to stand and forming a cell structure which includes the biocompatible macromolecular block and the cell in the recessed portion and in which the plurality of biocompatible macromolecular blocks are disposed in gaps between the plurality of cells; and

a step (C) of stirring and culturing a content of the first culture container obtained in the step (B) in a second culture container comprising stirring means

(i) wherein the first culture container is allowed to stand in the step (B) for a period of time for which a ratio of the number of cell structures manufactured after the step (C) to the number of recessed portions is 70% or more,

(ii) wherein the recessed portions are formed by 10 recessed portions/cm2 to 10,000 recessed portions/cm2 per unit area of the well forming area (24), and

(iii) wherein the cells to be used are one or more selected from the group consisting of ES cells, iPS cells, MSCs, chondrocytes, osteoblasts, osteoprecursor cells, mesenchymal cells, myoblasts, cardiac muscle cells, cardiomyoblasts, nerve cells, hepatocytes, beta cells, fibroblasts, corneal endothelial cells, vascular endothelial cells, corneal epithelial cells, amniotic cells, umbilical cord blood cells, bone marrow derived cells, and hematopoietic stem cells.

In the manufacturing method according to the embodiment of the present invention, it is possible to add the mixture of the biocompatible macromolecular blocks, the cells, and the liquid medium, to the first culture container having the culture surface on which the plurality of recessed portions are formed and a side wall part standing on the outer periphery of the culture surface such that the liquid surface of the mixture is over the culture surface, and adding the biocompatible macromolecular block, the cell, the liquid medium to all of the recessed portions of the first culture container in one step. According to the method of the embodiment of the present invention, a cell structure can be manufactured in a short period of time.

According to the present invention, in the recessed portion of the first culture container, a cell structure which includes biocompatible macromolecular blocks and cells and in which the plurality of biocompatible macromolecular blocks are disposed in gaps between the plurality of cells, and then a content of the first culture container is stirred and cultured in the second culture container comprising the stirring means. With respect to the cell structure obtained after the stirring culture step, for example, at least one of a ratio, a shape, or a size of the macromolecular block having biocompatibility are uniform. The expression "at least one of a ratio, a shape, or a size are uniform" does not mean that at least one of the ratio, the shape, or the size are completely the same, but means that at least one of the ratio, the shape, or the size are more uniform than those in a case where a cell structure is manufactured under conditions provided in Comparative Examples 1 to 3 below.

In the present invention, in a case where the cell structure is stirred and cultured, sufficient nutrient and oxygen can be delivered to the cells in the cell structure together with preventing fusion and disintegration of the cell structures, and thus it is possible to manufacture a cell structure having excellent cell viability.

In a case where the mixture of the biocompatible macromolecular blocks, the cells, and the liquid medium is added such that the liquid surface of the mixture is over the culture surface of the first culture container, the macromolecular block and the cell in which a cell structure is not formed on the upper surface between the recessed portions of the culture surfaces are present. Even in this case, by stirring and culturing the cell structure, the macromolecular block and the cell that do not form the cell structure are introduced into the cell structure, so as to form a larger cell structure. The physical force of stirring strengthens the adhesion between the macromolecular block and the cell which are included in the cell structure.

As above, in the method as defined in the claims, by applying a configuration of adding the mixture of the biocompatible macromolecular blocks, the cells, and the liquid medium to the first culture container such that the liquid surface of the mixture is over the culture surface, and stirring and culturing of the content of the first culture container after the cell structure is formed in the second culture container comprising the stirring means, it is possible to manufacture the cell structure uniformly and in large quantities for a short period of time.

(1) First culture container

The first culture container used in the present invention is a culture container having a culture surface on which a plurality of recessed portions are formed and a side wall part standing on an outer periphery of the culture surface.

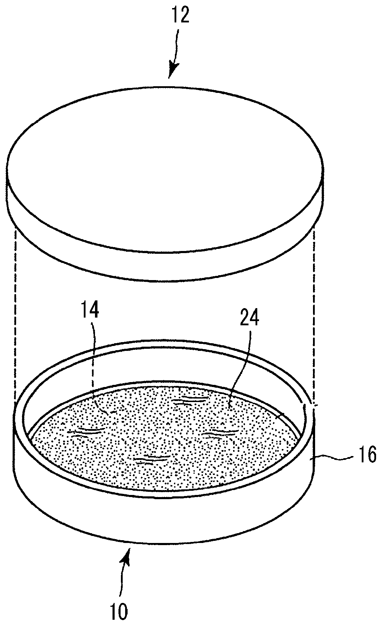

An example of the first culture container used in the present invention will be described with reference to Figs. 1 to 6.

In an example illustrated in Fig. 1, the culture container has a container main body 10 and a lid 12.

The container main body 10 has the disk-like bottom plate portion 14 and an annular side wall portion 16. The bottom plate portion 14 may be constituted with a synthesis resin material such as polystyrene or glass. For example, the bottom plate portion 14 can be manufactured through injection molding using the synthetic resin material.

The shape of the container may be a shape other than a disk shape or may be a shape such as a square. The side wall portion 16 rises from an outer peripheral edge of the bottom plate portion 14. The diameter of the bottom plate portion 14 can be set, for example, to 30 mm to 500 mm, the thickness of the bottom plate portion 14 can be set, for example, to 0.5 mm to 10 mm, and the height of the side wall portion 16 can be set, for example, to 20 mm to 100 mm, but are not particularly limited.

The lid 12 is formed in a shape corresponding to an opening portion on an upper portion of the container main body 10. The lid 12 can be used by covering the container main body 10 so as to maintain the culture environment of the cell.

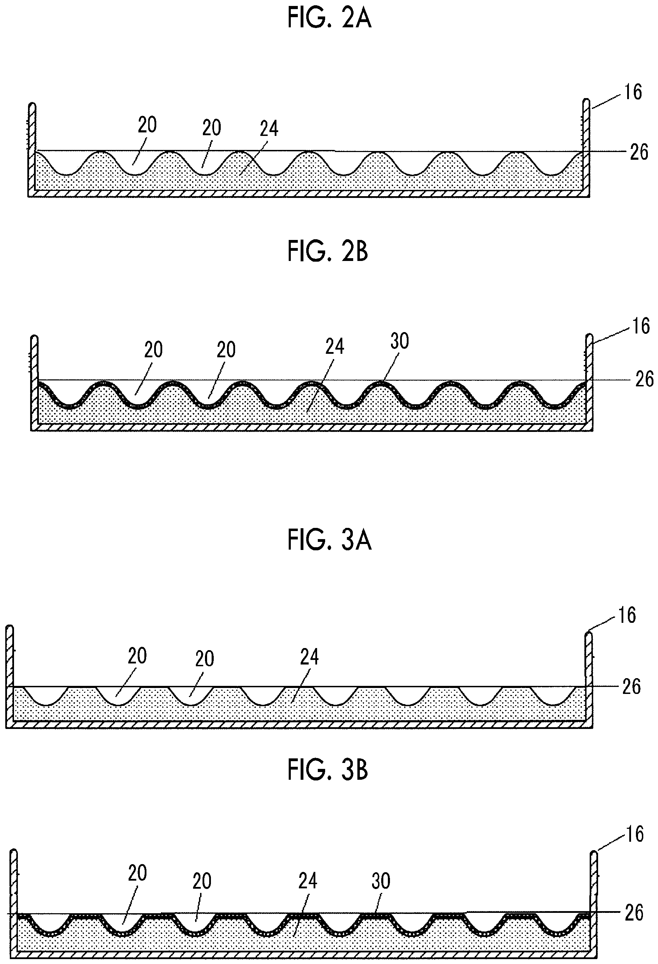

As illustrated in Figs. 2 and 3, a plurality of recessed portions 20 are formed in a well forming area 24 (that is, an area in which a compartment in which a culture object is cultured is formed) on the upper surface of the bottom plate portion 14. The inner surfaces of the recessed portions 20 are smooth concave surfaces. The recessed portions 20 form compartments (wells) in which an object to be cultured is cultured.

In the examples illustrated in Figs. 2A and 3A, a cell adhesion suppressant layer does not exist. In the examples illustrated in Figs. 2B and 3B, the cell adhesion suppressant layer 30 is provided.

A culture surface 26 means a surface including a most upper portion of the well forming area 24 (with reference to Figs. 2 to 5).

The depth of the recessed portion is not particularly limited, and is preferably 2 to 100 times of the size of the biocompatible macromolecular block, more preferably 2 to 10 times of the size of the biocompatible macromolecular block, and even more preferably 3 to 10 times of the size of the biocompatible macromolecular block.

The depths of the recessed portions are not particularly limited, but are preferably 10 to 2,000 $\mu$m, more preferably 20 to 1,000 $\mu$m, even more preferably 30 to 700 $\mu$m, still even more preferably 50 to 500 $\mu$m, and most preferably 100 to 400 $\mu$m.

A diameter of the recessed portion is not particularly limited, but is preferably 2 to 100 times of the size of the biocompatible macromolecular block, more preferably 3 to 50 times of the size of the biocompatible macromolecular block, and even more preferably 5 to 10 times of the size of the biocompatible macromolecular block.

The diameter of the recessed portion is not particularly limited, and is preferably 10 to 2,000 $\mu$m, more preferably 50 to 1,500 $\mu$m, even more preferably 100 to 1,500 $\mu$m, even more preferably 200 to 1,000 $\mu$m, and most preferably 400 to 1,000 $\mu$m.

It is preferable to cause the depth and the diameter of the recessed portion is in the above range, in view of having excellent performances of maintaining the strength and the shape with respect to the relationship with the size of the cell.

In a case where the recessed portion has the depth and the diameter, all of the recessed portions of the culture container are not required to have the above depth and the diameter, and at least a portion of the recessed portions may have the above depth and the above diameter.

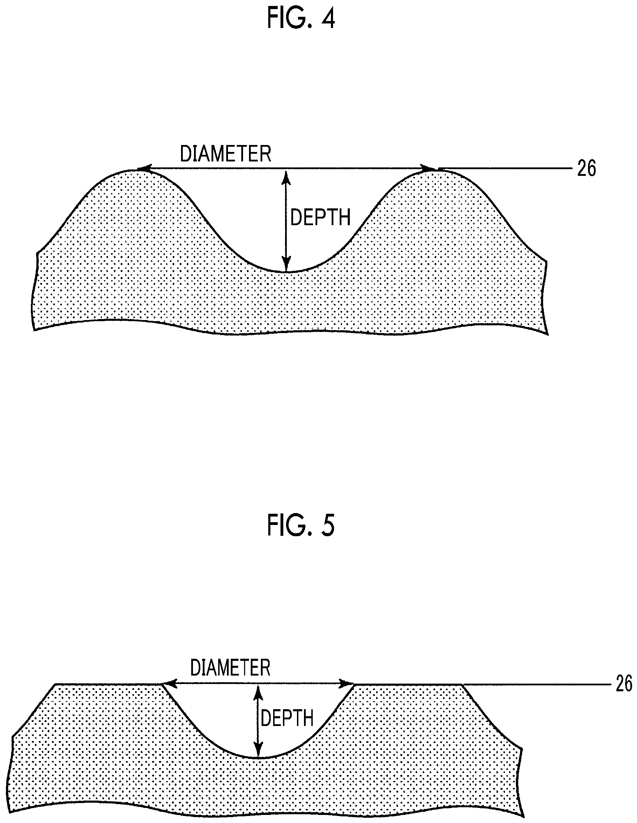

As illustrated in Figs. 4 and 5, the depth of the recessed portion means a gap between the most lower portion and the most upper portion of the recessed portion. As illustrated in Figs. 4 and 5, the diameter of the recessed portion means a length obtained by connecting the most upper portions of the recessed portions to each other. As shown in Fig. 5, in a case where the uppermost portions of the recessed portions are flat, the uppermost portions of recessed portions are selected so that the length connecting points of the uppermost portions of the recessed portions become the shortest.

The shape (including the depth and the diameter) of the recessed portion may be uniform or may not be uniform, but is preferably uniform. The depths and the diameters of the recessed portions are preferably uniform, and it is preferable that the depths and the diameters of all of the recessed portions are substantially the same.

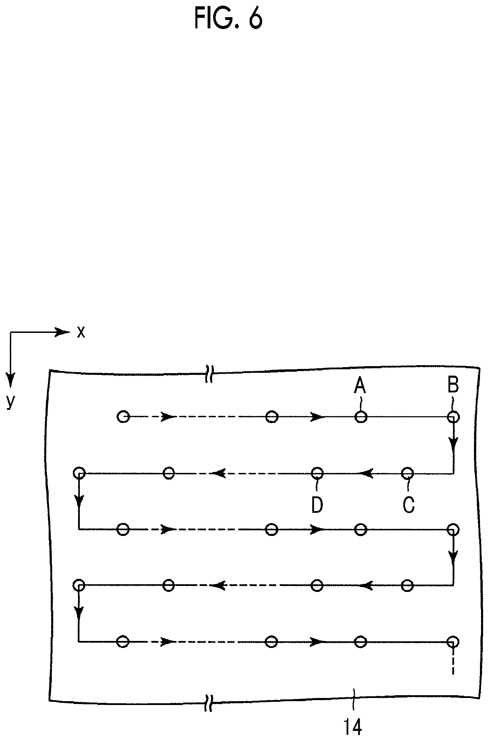

For example, the recessed portions 20 can be formed by irradiating the well forming area 24 with laser light. As shown in Fig. 6, laser irradiation is performed by irradiating the upper surface of the bottom plate portion 14 installed on the x-y plane with laser light in the z-axis direction.

First, a plurality of recessed portions 20 disposed in the x-axis direction are formed by emitting laser light at regular intervals (for example, 800 $\mu$m) while making an irradiation portion of a laser irradiation device to perform scanning in the positive direction of the x-axis. Subsequently, a plurality of recessed portions 20 disposed in the x-axis direction are formed by emitting laser light at regular intervals (for example, 800 $\mu$m) while the irradiation portion is made to perform scanning in the negative direction of the x-axis after the irradiation portion is made to perform scanning by a certain distance (for example, 400 $\mu$m) in the y-axis direction. Similarly, the irradiation portion is made to perform scanning by a certain distance (for example, 400 $\mu$m) in the y-axis direction. A plurality of recessed portions 20 regularly disposed on the upper surface of the bottom plate portion 14 are formed by repeating this process.

As shown in Fig. 6, in a case where the center coordinate (x, y) of an irradiation spot A is set to be an origin (0, 0), the center of an irradiation spot B close to the irradiation spot A is located at (0.8, 0), the center of an irradiation spot C is located at (0.4, 0.4), and the center of an irradiation spot D is located at (-0.4, 0.4). By shifting the x coordinate of the irradiation spots A and B and the x coordinate of the irradiation spots C and D in this manner, it is possible to densely form a plurality of recessed portions 20 in the well formation region 24. The recessed portions 20 are preferably formed by 10 recessed portions/$cm^2$ to 10,000 recessed portions/$cm^2$ per unit area of the well forming area 24. The number of the recessed portions is even more preferably 20 recessed portions/$cm^2$ to 8,000 recessed portions/$cm^2$, even more preferably 20 recessed portions/$cm^2$ to 3,000 recessed portions/$cm^2$, even more preferably 50 recessed portions/$cm^2$ to 1,000 recessed portions/$cm^2$, even more preferably 100 recessed portions/$cm^2$ to 500 recessed portions/$cm^2$, and particularly preferably 100 recessed portions/$cm^2$ to 300 recessed portions/$cm^2$.

As a laser light source, $CO_2$ laser is used, and the laser light can be used in the pulse irradiation at an output of 10 W and an irradiation speed of 6,100 mm/min.

Although the shapes of the irradiation spots are circular, the opening shapes of the recessed portions 20 are flattened in a substantially elliptical shape. It is considered that the flatness of the opening shapes is caused by the direction in which the synthetic resin material is poured into a metal mold during molding of the container main body 10.

In a case where the upper surface of the bottom plate portion 14 is irradiated with the laser light, the synthesis resin material constituting the bottom plate portion 14 is dissolved so as to form the recessed portions 20.

By adjusting the irradiation conditions such as the irradiation position and the output amount of the laser light, the distance between adjacent recessed portions 20, the diameters and depths of the recessed portions 20, the width and the height of the surface of the culture substrate between the recessed portions 20 adjacent to each other, and the like can be adjusted.

The well forming area 24 can be manufactured by using a die comprising protrusions forming the plurality of recessed portions 20 and recessed parts of forming the areas between recessed portions and injection molding the synthesis resin materials. The plurality of recessed portions 20 and the plurality of areas between recessed portions are formed together with the forming of the well forming area 24. In a case where the well forming area 24 is manufactured by using a die by injection molding, it is possible to form the recessed portions 20 with higher uniformity.

The area of the recessed portion is preferably 70% or more, more preferably 80% or more, even more preferably 90% or more, and most preferably 100% with respect to the total area of the culture surface. It is preferable to set the ratio of the area of the recessed portions to be within the above-described range from the viewpoint of the effect of the present invention.

The area of the recessed portions means the area in a case where the recessed portions are two-dimensionally caught in a case where the recessed portions are observed from above, and means the area of the region defined by the diameter of the recessed portion described above in the present specification. As illustrated in Figs. 2 or 4, in a case where a flat part is not present on the culture surface, the area of the recessed portion becomes 100% with respect to the total area of the culture surface.

Two recessed portions 20 adjacent to each other are formed via an area between the recessed portions. The surface of the culture substrate between the mutually adjacent recessed portions 20 may be flat or non-flat, but is preferably non-flat.

It is preferable that a treatment of suppressing cell adhesion is performed on the culture surface and the surface of the recessed portion. Accordingly, after the cell structure is cultured, the cell structure can be easily peeled off. Examples of the treatment of suppressing the cell adhesion include coating with a cell adhesion suppressant (see Figs. 2B and 3B). The cell adhesion suppressant plays a role of suppressing adhesion of cells to the upper surface of the bottom plate portion 14, particularly to the inner surfaces of the recessed portions 20. Examples of the cell adhesion suppressant include a phospholipid polymer, 2-methacryloyloxyethyl phosphoryl choline (MPC), polyhydroxyethyl methacrylate, or polyethylene glycol.

(2) Biocompatible macromolecular block

(2-1) Biocompatible macromolecules

Biocompatibility means a property which does not cause a significantly harmful reaction such as a long-term and chronic inflammatory reaction, during contact with a living body. Whether or not the biocompatible macromolecules used in the present invention are decomposed within a living body is not particularly limited as long as the biocompatible macromolecules have affinity to the living body. However, biodegradable macromolecules are preferable. Specific examples of non-biodegradable materials include polytetrafluoroethylene (PTFE), polyurethane, polypropylene, polyester, vinyl chloride, polycarbonate, acryl, stainless steel, titanium, silicone, and 2-methacryloyloxyethyl phosphorylcholine (MPC). Specific examples of the biodegradable material include a polypeptide (for example, gelatin described below) such as a naturally occurring peptide, a recombinant peptide, or a chemically synthesized peptide, polylactic acid, polyglycolic acid, a lactic acid/glycolic acid copolymer (PLGA), hyaluronic acid, glycosaminoglycan, proteoglycan, chondroitin, cellulose, agarose, carboxymethyl cellulose, chitin, and chitosan. Among these, a recombinant peptide is particularly preferable. Devising of an improvement of cell adhesion properties in these biocompatible macromolecules may be performed. Specifically, methods such as "Coating substrate surface with cell adhesion base material (fibronectin, vitronectin, and laminin) or a cell adhesion sequence (RGD sequence, LDV sequence, REDV sequence, YIGSR sequence, PDSGR sequence, RYVVLPR sequence, LGTIPG sequence, RNIAEIIKDI sequence, IKVAV sequence, LRE sequence, DGEA sequence, and HAV sequence, expressed as a single amino acid symbol)", "Animation and cationization of substrate surface", or "Plasma treatment of substrate surface and hydrophilic treatment by corona discharge" can be used.

The kinds of polypeptide including a recombinant peptide or a chemically synthesized peptide is not particularly limited, as long as the polypeptide has biocompatibility, but, for example, gelatin, collagen, elastin, fibronectin, pronectin, laminin, tenascin, fibrin, fibroin, entactin, thrombo spondin, and retronectin are preferable, and gelatin, collagen, and atelocollagen are most preferable. Gelatin for use in the present invention is preferably natural gelatin, recombinant gelatin, or chemically synthesized gelatin and more preferably recombinant gelatin. The natural gelatin referred to herein means gelatin manufactured using naturally derived collagen.

A chemically synthesized peptide or chemically synthesized gelatin means an artificially synthesized peptide or gelatin. The synthesis of a peptide such as gelatin may be solid phase synthesis or liquid phase synthesis, but is preferably solid phase synthesis. The solid phase synthesis of a peptide is well-known to those skilled in the art, and examples thereof include a fluorenyl-methoxy-carbonyl group (Fmoc group) synthesis method in which a Fmoc group is used for protection of an amino group, and a tert-butyl oxy carbonyl group (Boc group) synthesis method in which a Boc group is used for protection of an amino group. The contents described in (2-3) Gelatin below in the present specification can be applied to the preferable embodiments of the chemically synthesized gelatin.

Gelatin, particularly recombinant gelatin, is described below in the present specification.

A "1/IOB" value which is a hydrophilicity value of the biocompatible polymer used in the present invention is preferably 0 to 1.0. The value is more preferably within a range of 0 to 0.6, and even more preferably within a range of 0 to 0.4. IOB is an index of hydrophilic and hydrophobic properties based on an organic conceptual diagram representing polarity and non-polarity of an organic compound proposed by Atsushi HUJITA, and the details thereof are described in, for example, "Pharmaceutical Bulletin", vol. 2, 2, pp. 163-173 (1954), "Area of Chemistry" vol. 11, 10, pp. 719-725 (1957), and "Fragrance Journal, vol. 50, pp. 79-82 (1981). Briefly, the root of every organic compound is set to methane ($CH_4$), and all of other compounds are regarded as derivatives of methane. Certain numerical values for the number of carbons thereof, a substituent group, a transformation portion, a ring, and the like are set, and an organic value (OV) and an inorganic value (IV) are obtained by adding the score thereof. These values are plotted on a diagram in which the organic value is represented on the X-axis and the inorganic value is represented on the Y-axis. IOB in the organic conceptual diagram refers to a ratio of the inorganic value (IV) to the organic value (OV) in the organic conceptual diagram, that is, "inorganic value (IV)/organic value (OV)". The details of the organic conceptual diagram can be referred to "New Edition Organic Conceptual Diagram -Foundation and Application-" (written by Yoshio KOUDA, Sankyo Shuppan Co., Ltd., 2008). In the present specification, the hydrophilic and hydrophobic properties are represented by a "1/IOB" value which was obtained by taking a reciprocal number of IOB. This is a notation of representing more hydrophilic properties as the "1/IOB" value becomes small (close to 0).

In a case where the "1/IOB" value of the polymer used in the present invention is caused to be in the above range, hydrophilic properties become high, and water absorption properties become high, so as to effectively act on retention of nutritional components.

In a case where the biocompatible macromolecules used in the present invention are polypeptides, the hydrophilic and hydrophobic indexes represented by a grand average of hydropathicity (GRAVY) value are preferably -9.0 to 0.3, and more preferably -7.0 to 0.0. The grand average of hydropathicity (GRAVY) value can be obtained by methods of "Gasteiger E., Hoogland C., Gattiker A., Duvaud S., Wilkins M.R., Appel R.D., Bairoch A.; Protein Identification and Analysis Tools on the ExPASy Server; (In) John M. Walker (ed): The Proteomics Protocols Handbook, Humana Press (2005). pp. 571-607" and "Gasteiger E., Gattiker A., Hoogland C., Ivanyi I., Appeal R.D., Bairoch A.; ExPASy: the proteomics server for in-depth protein knowledge and analysis.; Nucleic Acids Res. 31:3784-3788 (2003)".

The hydrophilic properties and water absorbency become high by making the GRAVY value of the macromolecules used in the present invention be within the above-described range, which effectively acts to hold nutrient components.

(2-2) Cross-linking

The biocompatible macromolecules used in the present invention may be or may not be cross-linked, but are preferably cross-linked. By using the cross-linked biocompatible macromolecules, it is possible to obtain an effect of preventing instant decomposition during culturing in a medium and during transplantation into a living body. As general cross-linking methods, thermal cross-linking, cross-linking using aldehydes (for example, formaldehyde or glutaraldehyde), cross-linking using a condensation agent (carbodiimide, cyanamide, or the like), enzymatic cross-linking, photocrosslinking, ultraviolet cross-linking, a hydrophobic interaction, hydrogen bonding, an ionic interaction, and the like are known, it is also possible to use the above-described cross-linking methods in the method defined in the claims. As the cross-linking methods that may be used in the method defined in the claims, thermal cross-linking, ultraviolet cross-linking, or enzymatic cross-linking is more preferable, and thermal cross-linking is particularly preferable.

In a case where crosslinking by an enzyme is performed, the enzyme is not particularly limited, as long as the enzyme has a crosslinking action between macromolecular materials, but crosslinking can be performed preferably by using transglutaminase and laccase and most preferably by using transglutaminase. Specific examples of protein to be subjected to enzymatic cross-linking using transglutaminase are not particularly limited as long as the protein has a lysine residue and a glutamine residue. The transglutaminase may be derived from a mammal or a microorganism, and specific examples thereof include ACTIVA series manufactured by Ajinomoto Co., Inc., mammal-derived transglutaminase commercially available as a reagent, such as guinea pig liver-derived transglutaminase, goat-derived transglutaminase, and rabbit-derived transglutaminase manufactured by Oriental Yeast Co., Ltd., The Meridian Life Science, Inc., and the like, and human-derived blood coagulation factors (Factor XIIIa, Haematologic Technologies, Inc.).

The reaction temperature in a case of performing cross-linking (for example, thermal cross-linking) is not particularly limited as long as cross-linking can be performed, but is preferably -100°C to 500°C, more preferably 0°C to 300°C, even more preferably 50°C to 300°C, still even more preferably 100°C to 250°C, and still even more preferably 120°C to 200°C.

(2-3) Gelatin

The biocompatible macromolecules are preferably gelatin. The gelatin may be any of natural gelatin, recombinant gelatin, or chemically synthesized gelatin.

The recombinant gelatin referred in the present invention means polypeptides or protein-like substances which have an amino acid sequence similar to that of gelatin manufactured through gene recombination technology. The gelatin which can be used in the present invention preferably has a repeat of the sequence represented by Gly-X-Y specific to collagen (X and Y each independently represent any amino acid). Here, a plurality of Gly-X-Y's may be identical to or different from each other. Preferably, two or more sequences of cell adhesion signals are included in one molecule. As gelatin used in the present invention, gelatin having an amino acid sequence derived from a partial amino acid sequence of collagen can be used. Recombinant gelatins disclosed in, for example, EP1014176, US6992172B, WO2004/085473A, and WO2008/103041A can be used. However, the recombinant gelatin is not limited thereto. The gelatin used in the present invention is preferably gelatin according to an aspect below.

The recombinant gelatin is excellent in biocompatibility with original performance of natural gelatin, and is excellent in non-infection properties since there is no concern of bovine spongiform encephalopathy (BSE) and the recombinant gelatin with not being naturally derived. The recombinant gelatin is even compared to natural gelatin, and a sequence is determined. Therefore, it is possible to accurately design the strength and degradability so as to reduce deviation through cross-linking or the like.

The molecular weight of the gelatin is not particularly limited, but is preferably 2,000 to 100,000 (2 kDa to 100 kDa), more preferably 2,500 to 95,000 (2.5 kDa to 95 kDa), even more preferably 5,000 to 90,000 (5 kDa to 90 kDa), and most preferably 10,000 to 90,000 (10 kDa to 90 kDa).

The gelatin preferably has a repeat of the sequence represented by Gly-X-Y specific to collagen. Here, a plurality of Gly-X-Y's may be identical to or different from each other. In Gly-X-Y, Gly represents glycine and X and Y represent any amino acid (preferably represents any amino acid other than glycine). The sequence represented by Gly-X-Y characteristic to collagen is a partial structure which is extremely specific compared to other protein in a composition or a sequence of an amino acid of gelatin/collagen. In this section, glycine occupies about one-third of the entirety of the amino acid sequence, one sequence is repeated every three sequences. Glycine is the simplest amino acid. Therefore, there is a little restraint in disposition of molecular chains and glycine significantly contributes to regeneration of a helix structure during gelation. The amino acid represented by X and Y include a large amount of imino acid (proline and oxyproline), and preferably occupies 10% to 45% with respect to the total. Preferably 80% or more, more preferably 95% or more, and most preferably 99% or more of the amino acid of the gelatin sequence have the repeating structure of Gly-X-Y.

In general gelatin, a polar amino acid with an electrical charge and a polar non-charged amino acid exist by 1:1 in polar amino acids. Here, the polar amino acid specifically indicates cysteine, aspartic acid, glutamic acid, histidine, lysine, asparagine, glutamine, serine, threonine, tyrosine, or arginine. Among these, the polar non-charged amino acid indicates cysteine, asparagine, glutamine, serine, threonine, or tyrosine. In the gelatin used in the present invention, the proportion of the polar amino acids is 10% to 40% and preferably 20% to 30% with respect to the total constituting amino acid. It is preferable that the proportion of a non-charged amino acid in the polar amino acid is greater than or equal to 5% and less than 20% and preferably less than 10%. It is preferable not to include any one amino acid of serine, threonine, asparagine, tyrosine, or cysteine, preferably two or more of the amino acids in the sequence.

In general, in polypeptides, minimum amino acid sequences which work as cell adhesion signals are known (for example, Nagai Shoten Co., Ltd., "Pathophysiology", Vol. 9, No. 7 (1990) p. 527). The gelatin used in the present invention preferably has two or more of these cell adhesion signals in one molecule. As the specific sequences, sequences such as an RGD sequence, an LDV sequence, an REDV sequence, a YIGSR sequence, a PDSGR sequence, an RYVVLPR sequence, an LGTIPG sequence, an RNIAEIIKDI sequence, an IKVAV sequence, an LRE sequence, a DGEA sequence, and an HAV sequence, which are represented by one-letter notation of amino acids are preferable in that there are many kinds of cells adhered. An RGD sequence, a YIGSR sequence, a PDSGR sequence, an LGTIPG sequence, an IKVAV sequence, and a HAV sequence are more preferable and an RGD sequence is particularly preferable. In the RGD sequence, an ERGD sequence is preferable. By using gelatin having a cell adhesion signal, the amount of base material production of cells can be improved. For example, it is possible to improve the manufacturing of glycosaminoglycan (GAG) in a case of cartilage differentiation using mesenchymal stem cells as cells.

As the arrangement of RGD sequences in gelatin used in the present invention, the number of amino acids between RGDs is not uniform between 0 and 100 and preferably between 25 and 60.

The content of this minimum amino acid sequence is preferably 3 to 50, more preferably 4 to 30, and particularly preferably 5 to 20 in one molecule of protein in view of cell adhesion properties and proliferation properties. The most preferable content thereof is 12.

In the gelatin used in the present invention, the ratio of the RGD motif to the total number of amino acids is preferably at least 0.4%. In a case where the gelatin includes more than 350 amino acids, it is preferable that each stretch of 350 amino acids includes at least one RGD motif. The proportion of RGD motifs with respect to the total number of amino acids is even more preferably at least 0.6%, still even more preferably at least 0.8%, still even more preferably at least 1.0%, still even more preferably at least 1.2%, and most preferably at least 1.5%. The number of RGD motifs in the peptide is preferably at least 4, more preferably at least 6, more preferably at least 8, more preferably 12 or more and 16 or less per 250 amino acids. The proportion of RGD motifs being 0.4% corresponds to at least one RGD sequence per 250 amino acids. Since the number of RGD motifs is an integer, in order to satisfy the characteristics of at least 0.4%, gelatin including 251 amino acids is required to include at least used in two RGD sequences. The gelatin the present invention preferably includes at least 2 RGD sequences per 250 amino acids, more preferably at least 3 RGD sequences per 250 amino acids, and more preferably at least 4 RGDs per 250 amino used in acids. The gelatin the present invention includes at least 4 RGD motifs, preferably includes at least 6 RGD motifs, more preferably at least 8 RGD motifs, and even more preferably includes 12 to 16 RGD motifs.

The gelatin may be partially hydrolyzed.

It is preferable that the gelatin used in the present invention is represented by Formula 1: A-[(Gly-X-Y)$_n$]$_m$-B. n X's each independently represent any amino acid and n Y's each independently represent any amino acid. m preferably represents an integer of 2 to 10 and more preferably represents an integer of 3 to 5. n is preferably an integer of 3 to 100, more preferably an integer of 15 to 70, and most preferably an integer of 50 to 65. A represents any amino acid or an amino acid sequence, B represents any amino acid or an amino acid sequence. n Gly-X-Y's may be identical to or different from each other.

It is more preferable that the gelatin used in the present invention is represented by Formula: Gly-Ala-Pro-[(Gly-X-Y)$_{63}$]$_3$-Gly (in the formula, 63 X's each independently represent any of the amino acids, and 63 Y's independently represent any of the amino acids. 63 Gly-X-Y's may be identical to or different from each other).

It is preferable that a plurality of sequence units of collagen which naturally exists are bonded to a repeating unit. Any naturally existing collagen referred to herein may be used as long as the collagen naturally exists, but is preferably I type collagen, II type collagen, III type collagen, IV type collagen, or V type collagen, and more preferably I type collagen, II type collagen, or III type collagen. According to another form, the above-described collagen is preferably derived from a human-type, cattle, a pig, a mouse, or a rat, and is more preferably derived from a human-type.

The isoelectric point of the gelatin used in the present invention is preferably 5 to 10, more preferably 6 to 10, and even more preferably 7 to 9.5. The isoelectric point of the gelatin is measured by measuring pH after a 1 mass% gelatin solution is caused to pass through the mixed crystal column of an exchange resin by an isoelectric focusing method (see Maxey, C. R. (1976; Phitogr. Gelatin 2, Editor Cox, P. J. Academic, London, Engl.).

It is preferable that the gelatin is not deaminated.

It is preferable that the gelatin does not have telopentide.

It is preferable that the gelatin is a substantially pure polypeptide prepared by a nucleic acid coding an amino acid sequence.

The gelatin used in the present invention is particularly preferably

(1) a peptide formed of an amino acid sequence described in SEQ ID No: 1;
(2) a peptide which is formed of an amino acid sequence in which one or several amino acids are deleted, substituted, or added in the amino acid sequence described in SEQ ID No: 1, and has biocompatibility; or
(3) a peptide which is formed of an amino acid sequence having 80% or more (more preferably 90% or more, particularly preferably 95% or more, and most preferably 98% or more) sequence identity to the amino acid sequence described in SEQ ID No: 1, and has biocompatibility.

"One or several" in the expression "amino acid sequence in which one or several amino acids are deleted, substituted, or added" preferably means 1 to 20 amino acids, more preferably means 1 to 10 amino acids, even more preferably means 1 to 5 amino acids, and particularly preferably means 1 to 3 amino acids.

The recombinant gelatin used in the present invention can be manufactured through gene recombination technology which is known to those skilled in the art, and can be manufactured in accordance with, for example, methods disclosed in EP1014176A2, US6992172B, WO2004/085473A, and WO2008/103041A. Specifically, a gene encoding an amino acid sequence of predetermined recombinant gelatin is acquired, the acquired gene is incorporated into an expression vector to manufacture a recombinant expression vector, and a transformant is manufactured by introducing the recombinant expression vector into an appropriate host. The recombinant gelatin is manufactured by culturing the obtained transformant in an appropriate medium. Therefore, it is possible to prepare the gelatin used in the present invention by collecting the recombinant gelatin manufactured from a culture product.

(2-4) Biocompatible macromolecular block

In the present invention, a block (aggregation) formed of the above-described biocompatible macromolecules is used.

The shape of the biocompatible macromolecular block used in the present invention is not particularly limited. Examples thereof include an amorphous shape, a spherical shape, a particulate shape (granule), a powdery shape, a porous shape, a fibrous shape, a spindle shape, a flat shape, and a sheet shape. An amorphous shape, a spherical shape, a particulate shape (granule), a powdery shape, and a porous shape are preferable. The amorphous shape indicates that the shape of a surface is uneven, and indicates, for example, an object, such as rock, which has roughness. Examples of the above-described shapes are not distinct from each other. For example, in some cases, an example of a subordinate concept of the particulate shape (granule) is an amorphous shape.

The shape of the biocompatible macromolecular block used in the present invention is not particularly limited as described above. However, the tap density is preferably 10 mg/cm$^3$ to 500 mg/cm$^3$, more preferably 20 mg/cm$^3$ to 400 mg/cm$^3$, even more preferably 40 mg/cm$^3$ to 220 mg/cm$^3$, and particularly preferably 50 mg/cm$^3$ to 150 mg/cm$^3$.

The tap density is a value indicating how much volume of block can be densely filled. It can be seen that, as the value becomes smaller, the block cannot be densely filled, that is, the structure of the block is complicated. It is considered that the tap density of the biocompatible macromolecular block indicates the complexity of a surface structure of the biocompatible macromolecular block and the amount of void formed in a case where biocompatible macromolecular blocks are collected as an aggregate. As the tap density becomes smaller, the void between biocompatible macromolecular blocks becomes larger and a grafted region of a cell becomes larger. In addition, in a case where the tap density is not too small, the biocompatible macromolecular block can appropriately exist between cells and nutrients can be delivered into a cell structure in a case where the cell structure is manufactured, and therefore, it is considered that it is preferable that the tap density falls within the above-described range.

The tap density referred to in the present specification can be measured as follows. A container (with a cylindrical shape with a diameter of 6 mm and a length of 21.8 mm: a capacity of 0.616 cm$^3$) (hereinafter, described as a cap) is prepared for the measurement of the tap density. First, the mass of only a cap is measured. Thereafter, a funnel is attached to the cap, and blocks are poured from the funnel so as to be collected in the cap. After placing a sufficient amount of block, the cap portion is hit 200 times on a hard object such as a desk, the funnel is removed, and the blocks are leveled with a spatula. The mass is measured in a state where the cap is filled up with the blocks. The tap density can be obtained by calculating the mass of only the blocks from the difference between the mass of the cap filled up with the blocks and the mass of only the cap, and dividing the mass of only the blocks by the volume of the cap.

The cross-linking degree of the biocompatible macromolecular block used in the present invention is not particularly limited, but is preferably greater than or equal to 2, more preferably 2 to 30, even more preferably 4 to 25, and particularly preferably 4 to 22.

The method for measuring the solid (the number of cross-linking times per molecule) of a biocompatible macromolecular block is not particularly limited. However, the cross-linking degree can be measured, for example, through a TNBS (2,4,6-trinitrobenzene sulfonic acid) method in examples to be described below. Specifically, a sample obtained by mixing biocompatible macromolecular blocks, a $NaHCO_3$ aqueous solution, and a TNBS aqueous solution, allowing the mixture to react for 3 hours at 37°C, and then, stopping the reaction, and a blank obtained by mixing biocompatible macromolecular blocks, a $NaHCO_3$ aqueous solution, and a TNBS aqueous solution and stopping a reaction immediately after the mixing were prepared. The cross-linking degree (the number of cross-linking times per molecule) can be calculated from (Formula 2) and (Formula 3) by measuring each absorbance (345 nm) of the sample and the blank which have been diluted with pure water.

$$\text{(Formula 2)}(As - Ab) / 14{,}600 \times V / w$$

(Formula 2) represents the amount (molar equivalent) of lysine per 1 g of biocompatible macromolecular blocks.
(in the formula, As represents the sample absorbance, Ab represents the blank absorbance, V represents the amount (g) reaction liquid, and w represents the mass (mg) of the biocompatible macromolecular blocks.)

$$\text{(Formula 3)}1 - (\text{sample (Formula 2)} / \text{uncross-linked macromolecules (Formula 2)}) \times 34$$

(Formula 3) represents the number of cross-linking times per molecule.
The water absorption rate of the biocompatible macromolecular block used in the present invention is not particularly limited, but is preferably greater than or equal to 300%, more preferably greater than or equal to 400%, even more preferably greater than or equal to 500%, particularly preferably greater than or equal to 700%, and most preferably greater than or equal to 800%. The upper limit of the water absorption rate is not particularly limited, but is generally less than or equal to 4,000% or less than or equal to 2,000%.
The method for measuring the water absorption rate of the biocompatible macromolecular block is not particularly limited. However, the water absorption rate of the biocompatible macromolecular block can be measured, for example, through the method in examples to be described below. Specifically, a 3 cm × 3 cm nylon mesh bag is filled with about 15 mg of biocompatible macromolecular blocks at 25°C and is swollen in ion exchange water for 2 hours. Then, the biocompatible macromolecular blocks are dried with air for 10 minutes, and the mass is measured at each stage to obtain the water absorption rate according to (Formula 4).

$$\text{(Formula 4)}$$
$$\text{Water absorption rate} = (w2 - w1 - w0) / w0$$

(in the formula, w0 represents the mass of a material before water absorption, w1 represents the mass of an empty bag after water absorption, and w2 represents the mass of the entirety of the bag containing the material after water absorption.)
The size of one biocompatible macromolecular block according to the embodiment of the present invention is not particularly limited, but is preferably 1 μm to 700 μm, more preferably 10 μm to 700 μm, even more preferably 10 μm to 300 μm, still even more preferably 20 μm to 200 μm, still even more preferably 20 μm to 150 μm, and particularly preferably 53 μm to 106 μm. It is possible to favorably deliver nutrients into a cell structure from the outside by setting the size of one biocompatible macromolecular block to be within the above-described range. The size of one biocompatible macromolecular block does not mean that an average value of the sizes of a plurality of biocompatible macromolecular blocks is within the above-described range, but means the size of each biocompatible macromolecular block which is obtained by sieving a plurality of biocompatible macromolecular blocks.
The size of one block can be defined by the size of a sieve used in a case of dividing the block. For example, blocks remaining on a sieve with 106 μm in a case where blocks which have been passed through a sieve with 180 μm for sifting are sifted using the sieve with 106 μm can be regarded as blocks having a size of 106 to 180 μm. Next, blocks remaining on a sieve with 53 μm in a case where blocks which have been passed through the sieve with 106 μm for sifting are sifted using the sieve with 53 μm can be regarded as blocks having a size of 53 to 106 μm. Next, blocks remaining on a sieve with 25 μm in a case where blocks which have been passed through the sieve with 53 μm for sifting are sifted using the sieve with 25 μm can be regarded as blocks having a size of 25 to 53 μm.

(2-5) Method for manufacturing biocompatible macromolecular block

The method for manufacturing a biocompatible macromolecular block is not particularly limited. For example, it is possible to obtain a biocompatible macromolecular block by pulverizing a solid matter (such as a porous body of a biocompatible macromolecule) containing a biocompatible macromolecule using a pulverizer (such as NEW POWERMILL). The solid matter (such as a porous body of a biocompatible macromolecule) containing a biocompatible macromolecule can be obtained, for example, by freeze-drying an aqueous solution containing the biocompatible macromolecule.

It is possible to produce an amorphous biocompatible macromolecular block of which the shape of the surface is uneven, by pulverizing a solid matter containing a biocompatible macromolecule as described above.

An example of the method for manufacturing a porous body of a biocompatible macromolecule include a method including

(a) a step of cooling a solution of biocompatible macromolecules under the conditions where the difference between the temperature of a portion having the highest liquid temperature within the solution and the temperature of a portion having the lowest liquid temperature within the solution is lower than or equal to 2.5°C and the temperature of a portion having the highest liquid temperature within the solution is lower than or equal to a melting point, to an unfrozen state,

(b) a step of freezing the solution of the biocompatible macromolecules obtained in the step (a), and

(c) a step of freeze-drying the frozen biocompatible macromolecules obtained in the step (b).

In a case where the solution of the biocompatible macromolecules is cooled to an unfrozen state, the variation in the size of obtained porous pores is reduced by making the difference between the temperature of a portion having the highest liquid temperature in the solution and the temperature of a portion having the lowest liquid temperature in the solution be lower than or equal to 2.5°C (preferably lower than or equal to 2.3°C and more preferably lower than or equal to 2.1°C), that is, by reducing the difference in temperature. The lower limit of the difference between the temperature of a portion having the highest liquid temperature in the solution and the temperature of a portion having the lowest liquid temperature in the solution is not particularly limited, but may be higher than or equal to 0°C. For example, the lower limit thereof may be higher than or equal to 0.1°C, higher than or equal to 0.5°C, higher than or equal to 0.8°C, or higher than or equal to 0.9°C.

The cooling in the step (a) is preferably carried out, for example, using a material (preferably TEFLON (registered trademark)) having a lower thermal conductivity than water. The portion having the highest liquid temperature within the solution can be supposed as the farthest portion from a cooling side, and the portion having the lowest liquid temperature within the solution can be supposed as a liquid temperature of the cooled surface.

In the step (a), the difference between the temperature of a portion having the highest liquid temperature within the solution and the temperature of a portion having the lowest liquid temperature within the solution immediately before generation of solidification heat is preferably lower than or equal to 2.5°C, more preferably lower than or equal to 2.3°C, and even more preferably lower than or equal to 2.1°C. Here, the "difference in temperature immediately before the generation of solidification heat" means a difference in temperature in a case where the difference in temperature becomes largest between 1 second and 10 seconds before the generation of solidification heat.

In the step (a), the temperature of a portion having the lowest liquid temperature within the solution is preferably lower than or equal to a melting point of a solvent -5°C, more preferably lower than or equal to a melting point of a solvent -5°C and higher than or equal to a melting point of a solvent -20°C, and even more preferably lower than or equal to a melting point of a solvent -6°C and higher than or equal to a melting point of a solvent -16°C. The solvent of a melting point of a solvent is a solvent of a solution of biocompatible macromolecules.

In the step (b), the solution of the biocompatible macromolecules obtained in the step (a) is frozen. The cooling temperature for the freezing in the step (b) is not particularly limited. Depending on cooling equipment, the cooling temperature is preferably a temperature which is 3°C to 30°C lower than the temperature of a portion having the lowest liquid temperature within the solution, more preferably a temperature which is 5°C to 25°C lower than the temperature of a portion having the lowest liquid temperature within the solution, and even more preferably a temperature which is 10°C to 20°C lower than the temperature of a portion having the lowest liquid temperature within the solution.

In the step (c), the frozen biocompatible macromolecules obtained in the step (b) are freeze-dried. The freeze-drying can be performed through a usual method. For example, the freeze-drying can be performed by carrying out vacuum drying at a temperature lower than a melting point of a solvent and further carrying out vacuum drying at room temperature (20°C).

A biocompatible macromolecular block can be preferably manufactured by pulverizing the porous body obtained in the above-described step (c).

(3) Cell

As the cells used in the present invention one type of cell may be used, or a plurality of types of cells may be used in combination. It is possible to use, for example, embryonic stem (ES) cells or artificial pluripotent stem (iPS) cells as the universal cells. It is possible to use, for example, mesenchymal stem cells (MSC), hematopoietic stem cells, amniotic cells, umbilical cord blood cells, bone marrow-derived cells can be used as the somatic stem cell. It is possible to use, for example, ES cells, iPS cells, MSCs, chondrocytes, osteoblasts, osteoprecursor cells, mesenchymal cells, myoblasts, cardiac muscle cells, cardiomyoblasts, nerve cells, hepatocytes, beta cells, fibroblasts, corneal endothelial cells, vascular endothelial cells, corneal epithelial cells, amniotic cells, umbilical cord blood cells, bone marrow derived cells, or hematopoietic stem cells as the human-type derived cells. The cells may be derived from any of autologous cells and heterologous cells.

For example, it is possible to suitably use, for example, cardiac muscle cells, fibroblasts, and bone marrow cells (particularly, bone marrow cells differentiated into myocardial-like cells) which are autologous. Furthermore, cells for use in transplantation can be appropriately selected in other organs.

In addition, examples of the cells for use in cell transplantation include cells for use in cell transplantation for diabetic organ disorders. For example, there are cells for use in cell transplantation therapy in which diseases such as blood circulation disorders in the kidney, the pancreas, peripheral nerves, the eyes, and the limbs are intensively studied. That is, attempts to transplant insulin-secreting cells into the pancreas with a decreased insulin secretion ability, transplantation of bone marrow-derived cells into limbs with circulatory disorders, and the like are have been studied, and such cells can be used.

In the present invention, vascular cells can also be used. In the present specification, the vascular cells mean cells associated with angiogenesis, and are cells forming blood vessels and blood or precursor cells capable of being differentiated into the cells, or somatic stem cells. Here, cells such as mesenchymal stem cells (MSC) or universal cells such as ES cells, GS cells, or iPS cells, which are not naturally differentiated into cells forming blood vessels and blood are not included in the vascular cells. The vascular cells are preferably cells forming a blood vessel. In vertebrate-derived cells (particularly, human-derived cells), specific examples of the cells forming blood vessels include vascular endothelial cells and vascular smooth muscle cells. The vascular endothelial cells may be either venous endothelial cells or arterial endothelial cells. Vascular endothelial precursor cells can be used as precursor cells of the vascular endothelial cells. Vascular endothelial cells and vascular endothelial precursor cells are preferably used. Blood cells can be used as the cells forming blood. It is possible to use white blood cells such as lymphocytes or neutrophils, monocyte cells, and hematopoietic stem cells which are stem cells thereof.

In the present specification, non-vascular cells mean cells other than the above-described vascular cells. For example, ES cells, iPS cells, mesenchymal stem cells (MSC), myocardial stem cells, cardiac muscle cells, fibroblasts, myoblasts, chondrocytes, myoblasts, hepatocytes or nerve cells can be used. MSC, chondrocytes, myoblasts, myocardial stem cells, cardiac muscle cells, hepatocytes, or iPS cells can be preferably used. MSC, myocardial stem cells, cardiac muscle cells, or myoblasts can be more preferably used.

(4) Method for manufacturing cell structure

In the step (A) of the method of manufacturing a cell structure according to the embodiment of the present invention, the mixture of the biocompatible macromolecular blocks, the cells, and the liquid medium is added to the first culture container having the culture surface on which the plurality of recessed portions are formed and the side wall part standing on the outer periphery of the culture surface, such that the liquid surface of the mixture is over the culture surface.

The kind of the liquid medium is not particularly limited, and can be appropriately selected according to the kinds of the used cells, but for example, the liquid medium may be a growth medium or a differentiation medium.

Examples of the proliferation medium include Dulbecco's Modified Eagle Medium (DMEM) + 10% fetal bovine serum (FBS), MSCGM BulletKit (trademark) of Takara Bio Inc. and an EGM-2 + ECFC serum supplement of Lonza, but is not particularly limited.

Examples of the differentiation medium include a mesenchymal stem cell chondrocyte differentiation medium (Mesenchymal Stem Cell Chondrogenic Differentiation Medium) of Takara Bio Inc., a mesenchymal stem cell osteoblast differentiation medium (Mesenchymal Stem Cell Osteogenic Differentiation Medium) of Takara Bio Inc., but is not particularly limited.

In the step (B) of the method of manufacturing a cell structure according to the embodiment of the present invention, a cell structure in which the first culture vessel of the step (A) is allowed to stand, biocompatible macromolecular blocks and cells are included in the recessed portion, and the plurality of biocompatible macromolecular blocks are disposed in gaps between the plurality of cells is formed.

In the step (B), the first culture container is allowed to stand until the free biocompatible macromolecular blocks are preferably 30 mass% or less (more preferably 10 mass% or less, even more preferably 5 mass% or less, and most preferably 0 mass%) with respect to the total biocompatible macromolecular blocks. A case where "the free biocompatible macromolecular block is 0 mass% of the total biocompatible macromolecular block" is a case where a free biocompatible macromolecular block is not present.

It is preferable that the first culture container is allowed to stand in the step (B) for a period of time for which the ratio of the number of cell structures manufactured after the step (C) to the number of recessed portions is 70% or more.

In the step (B), the period of time for allowing the first culture container to stand is preferably 2 hours to 24 hours and more preferably 3 hours to 16 hours. In a case where the standing time is 2 or more hours, the adhesion between the biocompatible macromolecular block and the cell becomes satisfactory, and thus in a case of being provided in the stirring culture step, the separation between the biocompatible macromolecular block and the cell can be suppressed, such that the cell structure can be effectively formed. In a case where the standing time is 24 hours or less, the fusion of cell structures can be suppressed, and thus a large number of uniform cell structures can be obtained.

The step (B) can be performed in a $CO_2$ incubator as desired, and is performed generally at 30°C to 45°C and preferably 35°C to 40°C (for example, 37°C).

In the step (C) of the method of manufacturing the cell structure according to the embodiment of the present invention, the content of the first culture container obtained in the step (B) is stirred and cultured in the second culture container comprising the stirring means.

The content of the first culture container includes a liquid medium, a cell structure, a biocompatible macromolecular block not forming the cell structure, and a cell not forming the cell structure.

An operation of transferring the content of the first culture container obtained in the step (B) to a second culture container comprising the stirring means is not particularly limited, but can be performed by a general method, and for example, the content of the first culture container can be transferred to a second culture container comprising stirring means by using a pippet.

The second culture container is a container of stirring and culturing and comprises stirring means. As the stirring means, a stirring blade, a stirrer, or means for rotating or vibrating the container can be used. As the second culture container, a commercially available culture container (for example, a product name of 30 mL single use bioreactor (BWV-S03A manufactured by ABLE Corporation)) can also be used.

As the medium used in the stirring and culturing in the step (C), the same medium as the liquid medium described in the step (A) can be used. The medium used in the stirring and culturing in the step (C) may be the same medium as the liquid medium used in the step (A) or may be a different medium, but is preferably the same medium. In a case where the medium used in the step (C) is the same liquid medium used in the step (A), in the step (C), stirring and culturing may be performed after the medium is changed into a new medium.

The period of time for stirring and culturing the content of the first culture container in the step (C) is preferably 12 hours to 93 hours and more preferably 18 hours to 48 hours. In a case where stirring and culturing may be performed for the above period of time, cell structures having uniform ratios, shapes, and sizes of the macromolecular blocks having biocompatibility and the cells can be easily manufactured.

The stirring speed in the stirring and culturing is preferably 20 to 200 rpm and more preferably 60 to 90 rpm. rpm refers to revolutions per minute, and 1 rpm = 1 $min^{-1}$.

(5) Cell structure

The cell structure manufactured through the method according to the embodiment of the present invention contains biocompatible macromolecular blocks and cells. In the present invention, a plurality of biocompatible macromolecular blocks are three-dimensionally disposed in gaps between a plurality of cells in a mosaic shape using the biocompatible macromolecular blocks and the cells. Accordingly, it is possible to deliver nutrients to the inside of the cell structure from the outside. In the present specification, in some cases, the cell structure may be referred to as a mosaic cell aggregation (a cell aggregation having a mosaic shape).

In at least a part of the cell structure, the plurality of biocompatible macromolecular blocks are disposed in gaps between the plurality of cells. Here, the "gaps between cells" is not necessarily a space closed by the constituent cells, and may be interposed by the cells. Gaps are not necessarily present between all of the cells, and there may be a place where cells are brought into contact with each other. The distance of gaps between cells through biocompatible macromolecular blocks, that is, the gap distance in a case of selecting a certain cell, and a cell existing in the shortest distance from the certain cell is not particularly limited. However, the distance is preferably the size of a biocompatible macromolecular block, and a favorable distance is also within the range of the favorable size of a biocompatible macromolecular block.

In addition, the biocompatible macromolecular blocks have a configuration of being interposed by the cells. However, there are not necessarily cells between all of the biocompatible macromolecular blocks, and there may be a place where biocompatible macromolecular blocks are brought into contact with each other. The distance between biocompatible macromolecular blocks through cells, that is, the distance in a case of selecting a biocompatible macromolecular block, and a biocompatible macromolecular block existing in the shortest distance from the biocompatible macromolecular block is not particularly limited. However, the distance is preferably the size of an aggregation of cells in a case where one or several cells to be used are gathered. For example, the size thereof is 10 μm to 1,000 μm, preferably 10 μm to 500 μm, and more preferably 10 μm to 200 μm.

In the cell structure, the ratio of a biocompatible macromolecular block to a cell is not particularly limited. However, it is preferable that the ratio of a biocompatible macromolecular block per cell is preferably 0.0000001 μg to 1 μg, more preferably 0.000001 μg to 0.1 μg, more preferably 0.00001 μg to 0.01 μg, and most preferably 0.00002 μg to 0.006 μg. By setting the ratio of the biocompatible macromolecular blocks to the cells to be within the above-described range, it is possible to make the cells more evenly exist. By setting the lower limit to be within the above-described range, it is possible to exhibit an effect of the cells in a case of using the cells for a desired purpose. Moreover, by setting the upper limit to be within the above-described range, it is possible to supply components in arbitrarily existing biocompatible macromolecular blocks to cells. Here, the components in biocompatible macromolecular blocks are not particularly limited, but examples thereof include components contained in a liquid medium.

The thickness or the diameter of the manufactured cell structure is preferably 10 μm to 1 cm or less, more preferably 10 μm to 2,000 μm, even more preferably 15 μm to 1,500 μm, and most preferably 20 μm to 1,300 μm. In a case where the thickness or the diameter of the cell structure is caused to be in the above range, nutrient delivery from the outside to the inside of the cell structure becomes more satisfactory.

In the cell structure manufactured according to the method as described in the claims, regions consisting of biocompatible macromolecular blocks and regions consisting of cells are preferably disposed in a mosaic shape. In the present specification, the expression "the thickness or the diameter of the cell structure" means the followings. In a case where one point A in the cell structure is selected, in a straight line passing through the point A, a length of the line segment dividing the cell structure such that the distance from an outer boundary of the cell structure is the shortest is set as a line segment A. In the cell structure, the point A at which the line segment A becomes the longest is selected, a length of the line segment A in this case refers to "a thickness or a diameter of a cell structure".

(6) Use of sheet-like cell structure

The sheet-like cell structure manufactured by the manufacturing method a as described in the claims can be for use in cell transplantation. Specifically, the sheet-like cell structure manufactured by the method as described in the claims can be for use in transplanting cells into sites with heart diseases such as severe heart failure and severe myocardial infarction and diseases such as cerebral ischemia and cerebral infarction. In addition, the cell structure manufactured by the method as described in the claims can also be for use in diabetic diseases such as blood circulation disorders in the kidney, the pancreas, the liver, peripheral nerves, the eyes, and the limbs.

The present invention will be more specifically described using the following examples;

Examples

[Reference Example 1] Recombinant Peptide (Recombinant Gelatin)

The following CBE3 (which is disclosed in WO2008/103041A) was prepared as recombinant peptides (recombinant gelatin).

CBE3:

Molecular weight: 51.6 kD
Structure: GAP[(GXY)$_{63}$]$_3$G
Number of amino acids: 571
RGD sequence: 12
Imino acid content: 33%
Almost 100% of amino acids have a repeating structure of GXY. In the amino acid sequence of CBE3, serine, threonine, asparagine, tyrosine, and cysteine are not included. CBE3 has an ERGD sequence.
Isoelectric point: 9.34
GRAVY value: -0.682
1/IOB value: 0.323
Amino acid sequence (SEQ ID No: 1 in a sequence table) (which is the same as that of SEQ ID No: 3 in WO2008/103041A. However, X in the end is corrected to "P").

GAP(GAPGLQGAPGLQGMPGERGAAGLPGPKGERGDAGPKGADGAPGAPGL
QGMPGERGAAGLPGPKGERGDAGPKGADGAPGKDGVRGLAGPIGPPGERGAAGLPG
PKGERGDAGPKGADGAPGKDGVRGLAGPIGPPGPAGAPGAPGLQGMPGERGAAGLP
GPKGERGDAGPKGADGAPGKDGVRGLAGPP)₃G

[Reference Example 2] Manufacturing Porous Body of Recombinant Peptide

[PTFE Thickness Cylindrical Container]

A cylindrical cup-shaped polytetrafluoroethylene (PTFE) container with a bottom surface thickness of 3 mm, a diameter of 51 mm, a side surface thickness of 8 mm, and a height of 25 mm was prepared. In a case where the curved surface of the cylindrical cup is set as a side surface, the side surface is closed by PTFE with 8 mm and the bottom surface (circular shape of a flat plate) is also closed by PTFE with 3 mm. In contrast, the upper surface is in an open shape. Accordingly, the inner diameter of the cylindrical cup is set to 43 mm Hereinafter, this container is referred to as a PTFE thickness cylindrical container.

[Aluminum Glass Plate Cylindrical Container]

A cylindrical cup-shaped aluminum container with a thickness of 1 mm and a diameter of 47 mm was prepared. In a case where the curved surface of the cylindrical cup is set as a side surface, the side surface is closed by aluminum with 1 mm and the bottom surface (circular shape of a flat plate) is also closed by aluminum with 1 mm. In contrast, the upper surface is in an open shape. In addition, TEFLON (registered trademark) with a thickness of 1 mm is evenly spread only in the inside of the side surface, and as a result, the inner diameter of the cylindrical cup becomes 45 mm. In addition, the bottom surface of this container enters a state where a 2.2 mm glass plate is joined to the bottom surface thereof on the outside of aluminum. Hereinafter, this container is referred to as an aluminum glass plate·cylindrical container.

[Freezing Step in which Difference in Temperature is Small, and Drying Step]

An aqueous CBE3 solution was made to flow into the PTFE thickness cylindrical container and the aluminum glass plate cylindrical container, and was cooled down from the bottom surface within a vacuum freeze dryer (TF5-85ATNNN: Takara Co., Ltd.) using a cooling shelf. A combination of the setting of the final concentration of the aqueous CBE3 solutions in the containers at this time, the amount of solution, and the temperature of the shelf was prepared as described below.

Condition A:

PTFE thickness cylindrical container, final concentration of aqueous CBE3 solution of 4 mass%, amount of aqueous solution of 4 mL. As the setting for the temperature of the shelf, the temperature was cooled down until the temperature reaches -10°C, and then, freezing was performed for 1 hour at -10°C, for 2 hours at -20°C, for 3 hours at -40°C, and finally for 1 hour at -50°C. Thereafter, the frozen product was subjected to vacuum drying for 24 hours at -20°C after the setting of the temperature of the shelf was returned to -20°C. After 24 hours, the temperature of the shelf was increased to 20°C in a state in which the vacuum drying was continued as it was, and the vacuum drying was further performed for 48 hours at 20°C until the vacuum degree was sufficiently decreased ($1.9 \times 10^5$ Pa). Then, the product was taken out of the vacuum freeze dryer. Accordingly, a porous body was obtained.

Condition B:

Aluminum glass plate cylindrical container, final concentration of aqueous CBE3 solution of 4 mass%, amount of aqueous solution of 4 mL. As the setting for the temperature of the shelf, the temperature was cooled down until the temperature reaches -10°C, and then, freezing was performed for 1 hour at -10°C, for 2 hours at -20°C, for 3 hours at -40°C, and finally for 1 hour at -50°C. Thereafter, the frozen product was subjected to vacuum drying for 24 hours at -20°C after the setting of the temperature of the shelf was returned to -20°C. After 24 hours, the temperature of the shelf was increased to 20°C in a state in which the vacuum drying was continued as it was, and the vacuum drying was further performed for 48 hours at 20°C until the vacuum degree was sufficiently decreased ($1.9 \times 10^5$ Pa). Then, the product was taken out of the vacuum freeze dryer. Accordingly, a porous body was obtained.

Condition C:

PTFE thickness cylindrical container, final concentration of aqueous CBE3 solution of 4 mass%, amount of aqueous solution of 10 mL. As the setting for the temperature of the shelf, the temperature was cooled down until the temperature reaches -10°C, and then, freezing was performed for 1 hour at -10°C, for 2 hours at -20°C, for 3 hours at -40°C, and finally for 1 hour at -50°C. Thereafter, the frozen product was subjected to vacuum drying for 24 hours at -20°C after the setting of the temperature of the shelf was returned to -20°C. After 24 hours, the temperature of the shelf was increased to 20°C in a state in which the vacuum drying was continued as it was, and the vacuum drying was further performed for 48 hours at 20°C until the vacuum degree was sufficiently decreased ($1.9 \times 10^5$ Pa). Then, the product was taken out of the vacuum freeze dryer. Accordingly, a porous body was obtained.

[Measurement of Temperature in Each Freezing Step]

Regarding the conditions A to C, the liquid temperature of the surface of water in a center portion of a circle within a container was measured as the liquid temperature (non-cooled surface liquid temperature) of the farthest portion from a cooling side in a solution, and the liquid temperature of a bottom portion within the container was measured as the liquid temperature (cooled surface liquid temperature) of the closest portion to the cooling side in the solution.

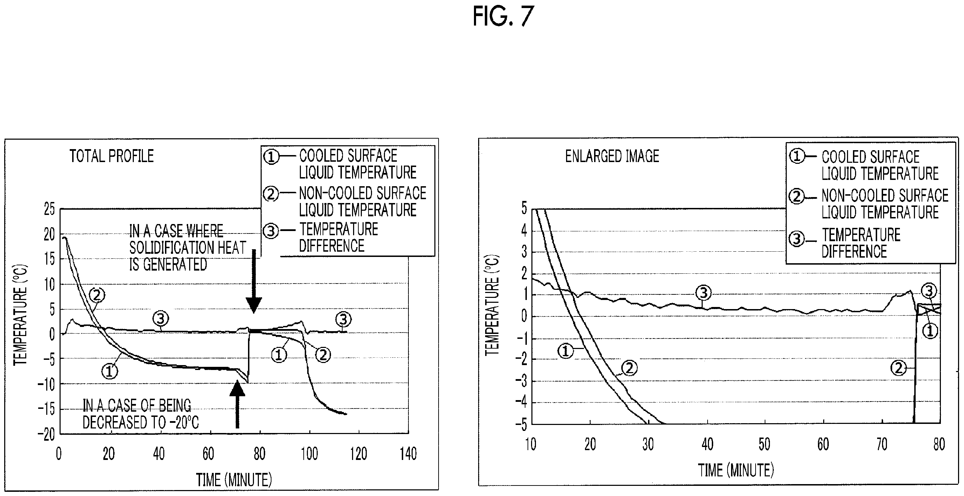

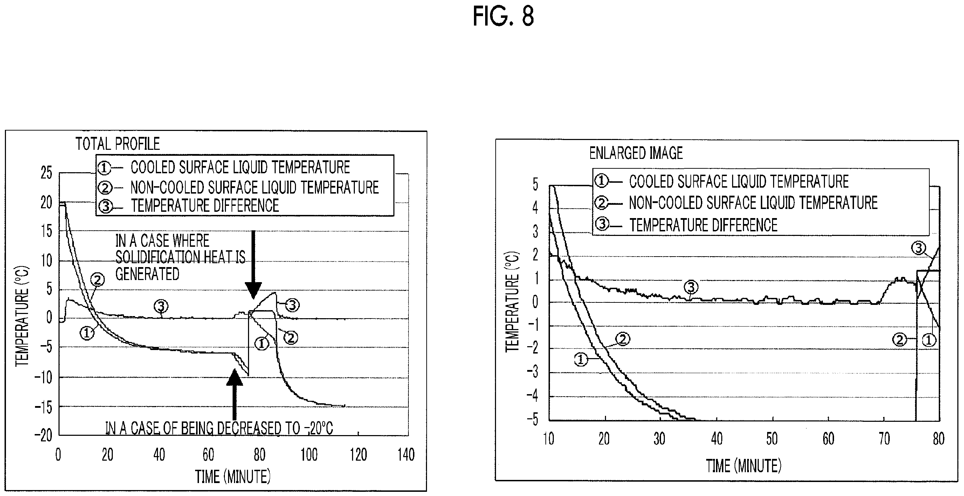

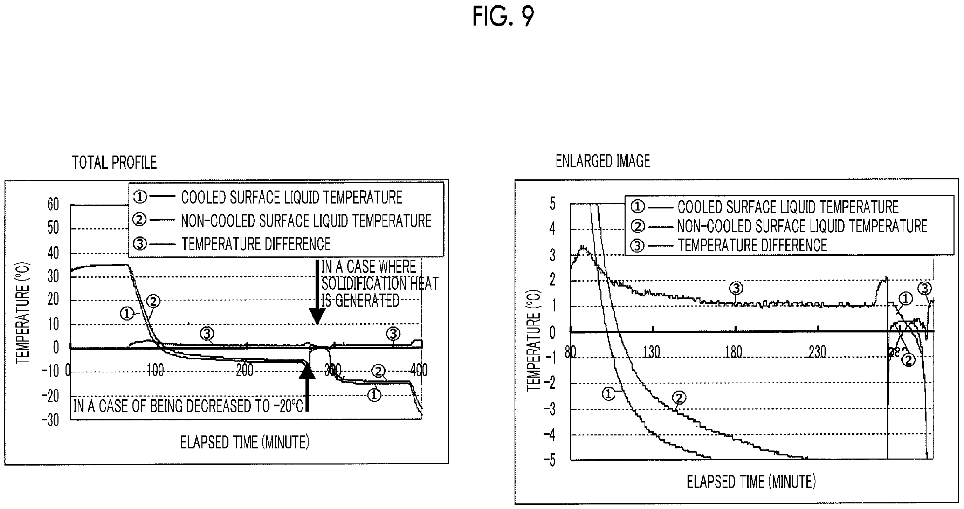

As a result, each temperature and a profile of the difference in temperature are as shown in Figs. 7 to 9.

It can be seen from Figs. 7 to 9 that the liquid temperature falls below 0°C, which is a melting point, in a setting section of the temperature of a shelf of -10°C (before the temperature decreases to -20°C) in the conditions A to C, and the solution enters a (unfrozen and overcooled) state where freezing does not occur in that state. In addition, in this state, the difference in temperature between the cooled surface liquid temperature and the non-cooled surface liquid temperature is less than or equal to 2.5°C. In the present specification, the "difference in temperature" means "non-cooled surface liquid temperature" - "cooled surface liquid temperature". Thereafter, the timing at which the liquid temperature rapidly rises to around 0°C by further lowering the temperature of the shelf to -20°C is confirmed. Here, it can be seen that freezing starts due to generation of solidification heat. In addition, it was also possible to confirm that ice formation actually started at the timing. Thereafter, the temperature was around 0°C while the certain time elapses. Here, the product entered a state where there was a mixture of water and ice. The temperature finally started to decrease again from 0°C, and at this time, the liquid portion is lost and becomes ice. Accordingly, the temperature being measured became a solid temperature within the ice, that is, was not the liquid temperature.

Hereinafter, regarding the conditions A to C, the difference in temperature at this time when the non-cooled surface liquid temperature became a melting point (0°C), the difference in temperature immediately before the temperature of the shelf is decreased from -10°C to -20°C, and the difference in temperature immediately before the generation of solidification heat will be described. The "difference in temperature immediately before" indicates the highest temperature in the difference in temperature which can be detected between 1 second to 20 seconds before an event (such as the generation of solidification heat).

Condition A:

Difference in temperature at this time when non-cooled surface liquid temperature became melting point (0°C): 1.1°C

Difference in temperature immediately before temperature is decreased from -10°C to -20°C: 0.2°C

Difference in temperature immediately before generation of solidification heat: 1.1°C

Condition B:

Difference in temperature at this time when non-cooled surface liquid temperature became melting point (0°C): 1.0°C

Difference in temperature immediately before temperature is decreased from -10°C to -20°C: 0.1°C

Difference in temperature immediately before generation of solidification heat: 0.9°C

Condition C:

Difference in temperature at this time when non-cooled surface liquid temperature became melting point (0°C): 1.8°C

Difference in temperature immediately before temperature is decreased from -10°C to -20°C: 1.1°C

Difference in temperature immediately before generation of solidification heat: 2.1°C

[Reference Example 3] Manufacturing Biocompatible Macromolecular Block (Pulverizing and Cross-Linking of Porous Body)

The CBE3 porous bodies of Conditions A and B which had been obtained in Reference Example 2 were pulverized using NEW POWERMILL (Osaka Chemical Co., Ltd., NEW POWERMILL PM-2005). The pulverizing was performed for one minute × 5 times, that is, for 5 minutes in total at the maximum rotation speed. The sizes of the obtained pulverized substances were divided using a stainless steel sieve to obtain uncross-linked blocks with 25 to 53 μm, 53 to 106 μm, and 106 to 180 μm. Thereafter, biocompatible macromolecular blocks (CBE3 blocks) were obtained by performing thermal cross-linking (six kinds of cross-linking times of 8 hours, 16 hours, 24 hours, 48 hours, 72 hours, and 96 hours) at 160°C under reduced pressure.

Hereinafter, a porous body derived block under the condition A which has been cross-linked for 48 hours is called E, and a porous body derived block under the condition B which has been cross-linked for 48 hours is called F. E and F are blocks with a small difference in temperature which have been manufactured from porous bodies manufactured through a freezing step in which the difference in temperature is small. There was no influence of the difference in cross-linking time on the performance in the evaluation of the present specification. Therefore, the blocks cross-linked for 48 hours were representatively used. There was no difference in performance between E and F. Hereinafter, the biocompatible macromolecular blocks obtained in Reference Example 3 are also referred to as "petal blocks". In reference examples, examples, and comparative examples, biocompatible macromolecular blocks which have sizes of 53 to 106 μm, are manufactured under the condition A, and of which the cross-linking time is 48 hours were used.

[Reference Example 4] Measurement of Tap Density of Biocompatible Macromolecular Block

The tap density is a value indicating how much volume of block can be densely filled. It can be said that, as the value becomes smaller, the block cannot be densely filled, that is, the structure of the block is complicated. The tap density was measured as follows. First, a funnel with an attached cap (having a cylindrical shape with a diameter of 6 mm and a length of 21.8 mm: capacity of 0.616 cm$^3$) at the tip thereof was prepared, and the mass of only the cap was measured. Thereafter, the cap was attached to the funnel, and blocks were poured from the funnel so as to be collected in the cap. After placing a sufficient amount of block, the cap portion was hit 200 times on a hard object such as a desk, the funnel was removed, and the blocks were leveled with a spatula. The mass was measured in a state where the cap was filled up with the blocks. The tap density was obtained by calculating the mass of only the blocks from the difference between the mass of the cap filled up with the blocks and the mass of only the cap, and dividing the mass of only the blocks by the volume of the cap.

As a result, the tap density of the biocompatible macromolecular blocks of Reference Example 3 was 98 mg/cm$^3$.

[Reference Example 5] Measurement of Cross-Linking Degree of Biocompatible Macromolecular Block

The cross-linking degree (the number of cross-linking times per molecule) of the blocks cross-linked in Reference Example 3 was calculated. The measurement was performed through a TNBS (2,4,6-trinitrobenzene sulfonic acid) method.

<Preparation of Sample>

A sample (about 10 mg), 4% NaHCO$_3$ aqueous solution (1 mL), and 1 mass% TNBS aqueous solution (2 mL) were added to a glass vial, and the mixture was shaken for 3 hours at 37°C. Thereafter, 37 mass% hydrochloric acid (10 mL) and pure water (5 mL) were added thereto, and then, the mixture was allowed to stand for 16 hours or longer at 37°C to prepare a sample.

<Adjustment of Blank>

A sample (about 10 mg), 4 mass% NaHCO$_3$ aqueous solution (1 mL), and 1 mass% TNBS aqueous solution (2 mL) were added to a glass vial, 37 mass% hydrochloric acid (3 mL) was immediately added thereto, and the mixture was shaken for 3 hours at 37°C. Thereafter, 37 mass% hydrochloric acid (7 mL) and pure water (5 mL) were added thereto, and then, the mixture was allowed to stand for 16 hours or longer at 37°C to prepare a blank.

The absorbance (345 nm) of the sample and the blank which had been diluted 10 times with pure water was measured, and the cross-linking degree (the number of cross-linking times per molecule) was calculated from (Formula 2) and (Formula 3).

$$(\text{Formula 2})(\text{As - Ab}) / 14{,}600 \times \text{V} / \text{w}$$

(Formula 2) represents the amount (molar equivalent) of lysine per 1 g of a recombinant peptide.

(in the formula, As represents the sample absorbance, Ab represents the blank absorbance, V represents the amount (g) reaction liquid, and w represents the mass (mg) of a recombinant peptide.)

(Formula 3)1 - (sample (Formula 2) / an uncross-linked recombinant peptide (Formula 2)) x 34

(Formula 3) represents the number of cross-linking times per molecule.

As a result, the cross-linking degree of the biocompatible macromolecular blocks of Reference Example 3 was 4.2.

[Reference Example 6] Measurement of Water Absorption Rate of Biocompatible Macromolecular Block

The water absorption rate of biocompatible macromolecular blocks manufactured in Reference Example 3 was calculated.

A 3 cm × 3 cm nylon mesh bag was filled with about 15 mg of the biocompatible macromolecular blocks at 25°C and was swollen in ion exchange water for 2 hours. Then, the biocompatible macromolecular blocks were dried with air for 10 minutes, and the mass was measured at each stage to obtain the water absorption rate according to (Formula 4).

$$(\text{Formula 4})$$

$$\text{Water absorption rate} = (\text{w2 - w1 - w0}) / \text{w0}$$

(in the formula, w0 represents the mass of a material before water absorption, w1 represents the mass of an empty bag after water absorption, and w2 represents the mass of the entirety of the bag containing the material after water absorption.)

As a result, the water absorption rate of the blocks of Reference Example 3 is 786%.

[Example 1] Manufacturing large number of cell structures (mosaic cell mass) (multi-well dish container + stirring culture step)

Human bone marrow-derived mesenchymal stem cells (hMSCs) were suspended in a proliferation medium (Takara Bio Inc.: MSCGM Bullet Kit (trademark)), and biocompatible macromolecular blocks (53 to 106 $\mu$m) prepared in Reference Example 3 were added thereto. The mixture was sown in EZSPHERE (registered trademark) DISH 90 mm Type 903 (which had a spheroid well diameter of 800 $\mu$m, a spheroid well depth of 300 $\mu$m, and about 6,000 spheroid wells, which was a cell non-adhesive dish, in a state in which hMSCs ($1.2 \times 10^8$ cells) and biocompatible macromolecular blocks (25 mg) were finally suspended in 23 mL of a medium, in which a bottom surface was a culture surface having a recess portion, and has a side outer wall erected on the periphery of the culture surface, and which was manufactured by AGC TECHNO GLASS CO., Ltd.). At this point, a mixture of biocompatible macromolecular blocks, cells, and a liquid medium was added to the 90 mm dish over the culture surface.

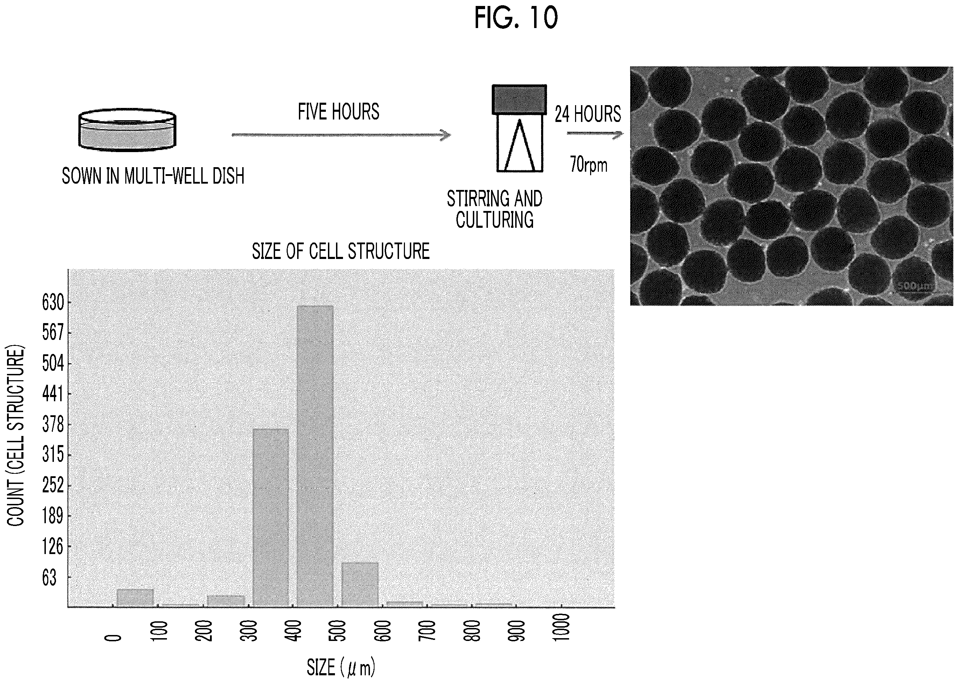

The above 90 mm dish was allowed to stand in a $CO_2$ incubator at 37°C for five hours (at this point, there was no free biocompatible macromolecular block). Thereafter, the entire contents were transferred to a product name of 30 mL single use bioreactor (BWV-S03A manufactured by ABLE Corporation) (second culture container comprising stirring means) by a pipette and allowed to stand for 10 minutes, a supernatant was removed and changed to 30 mL of a new medium, and stirring and culturing were performed at a rotation speed of 70 rpm for 24 hours. As a result, it was possible to successfully manufacture about 6,000 uniform cell structures without fusion and disintegration of the cell structures simultaneously. Manufacturing steps and results are illustrated in Fig. 10. As a result of measuring diameters of 1,169 cell structures, the minimum value was 23.89 $\mu$m, the maximum value was 909.40 $\mu$m, the average value was 419.51 $\mu$m, and the standard deviation was 625.81 $\mu$m.

[Example 2] Manufacturing large number of cell structures (mosaic cell mass) (multi-well dish container + stirring culture step)

Human bone marrow-derived mesenchymal stem cells (hMSCs) were suspended in a proliferation medium (Takara Bio Inc.: MSCGM Bullet Kit (trademark)), and biocompatible macromolecular blocks (53 to 106 $\mu$m) prepared in Reference Example 3 were added thereto. The mixture was sown in EZSPHERE (registered trademark) DISH 90 mm Type 903 (which had a spheroid well diameter of 800 $\mu$m, a spheroid well depth of 300 $\mu$m, and about 6,000 spheroid wells, which was a cell non-adhesive dish, in a state in which hMSCs ($1.2 \times 10^8$ cells) and biocompatible macromolecular blocks (25 mg) were finally suspended in 23 mL of a medium, in which a bottom surface was a culture surface having a recess portion, and has a side outer wall erected on the periphery of the culture surface, and which was manufactured by AGC TECHNO GLASS CO., Ltd.). At this point, a mixture of biocompatible macromolecular blocks, cells, and a liquid medium was added to the 90 mm dish over the culture surface.

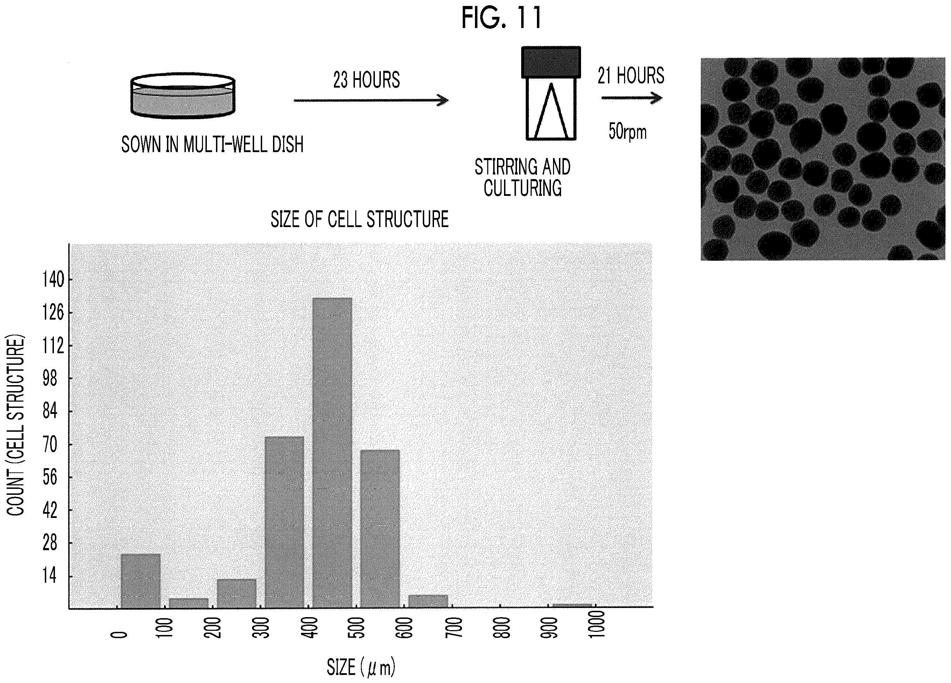

The above 90 mm dish was allowed to stand in a $CO_2$ incubator at 37°C for 23 hours (at this point, there was no free biocompatible macromolecular block). Thereafter, the entire contents were transferred to a product name of 30 mL single use bioreactor (BWV-S03A manufactured by ABLE Corporation) (second culture container comprising stirring means) by a pipette and allowed to stand for 10 minutes, a supernatant was removed and changed to 30 mL of a new medium, and stirring and culturing were performed at a rotation speed of 50 rpm for 21 hours. As a result, it was possible to successfully manufacture about 6,000 uniform cell structures without fusion and disintegration of the cell structures simultaneously. Manufacturing steps and results are illustrated in Fig. 11. As a result of measuring diameters of 317 cell structures, the minimum value was 23.89 μm, the maximum value was 953.95 μm, the average value was 415.56 μm, and the standard deviation was 498.80 μm.

[Comparative Example 1] Manufacturing large number of cell structures in multi-well dish container only (short-term culture)

Human bone marrow-derived mesenchymal stem cells (hMSCs) were suspended in a proliferation medium (Takara Bio Inc.: MSCGM Bullet Kit (trademark)), and biocompatible macromolecular blocks (53 to 106 μm) prepared in Reference Example 3 were added thereto. The mixture was sown in EZSPHERE (registered trademark) DISH 90 mm Type 903 (which had a spheroid well diameter of 800 μm, a spheroid well depth of 300 μm, and about 6,000 spheroid wells, and which was a cell non-adhesive dish, in a state in which hMSCs ($1.2 \times 10^8$ cells) and biocompatible macromolecular blocks (25 mg) were finally suspended in 23 mL of a medium, in which a bottom surface was a culture surface having a recess portion, and has a side outer wall erected on the periphery of the culture surface, and which was manufactured by AGC TECHNO GLASS CO., Ltd.). At this point, a mixture of biocompatible macromolecular blocks, cells, and a liquid medium was added to the 90 mm dish over the culture surface.

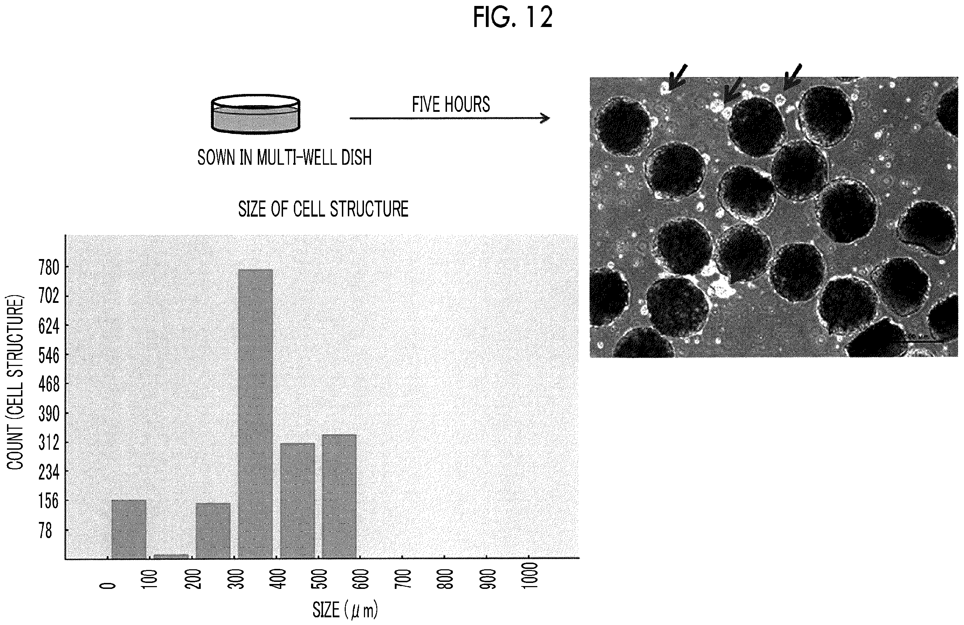

The above 90 mm dish was allowed to stand in a $CO_2$ incubator at 37°C for 5 hours and extracted, a large number of individual cells that do not form cell structures were checked, and shapes of the cell structures were not uniform. The results are provided in Fig. 12. It was found that it was not possible to obtain uniform cell structures only with this step. As a result of measuring diameters of 1,721 cell structures, the minimum value was 23.89 μm, the maximum value was 587.96 μm, the average value was 367.60 μm, and the standard deviation was 645.86 μm.

[Comparative Example 2] Manufacturing large number of cell structures in multi-well dish container only (long-term culture)

Human bone marrow-derived mesenchymal stem cells (hMSCs) were suspended in a proliferation medium (Takara Bio Inc.: MSCGM Bullet Kit (trademark)), and biocompatible macromolecular blocks (53 to 106 μm) prepared in Reference Example 3 were added thereto. The mixture was sown in EZSPHERE (registered trademark) DISH 90 mm Type 903 (which had a spheroid well diameter of 800 μm, a spheroid well depth of 300 μm, and about 6,000 spheroid wells, and which was a cell non-adhesive dish, in a state in which hMSCs ($1.2 \times 10^8$ cells) and biocompatible macromolecular blocks (25 mg) were finally suspended in 23 mL of a medium, in which a bottom surface was a culture surface having a recess portion, and has a side outer wall erected on the periphery of the culture surface, and which was manufactured by AGC TECHNO GLASS CO., Ltd.). At this point, a mixture of biocompatible macromolecular blocks, cells, and a liquid medium was added to the 90 mm dish over the culture surface.

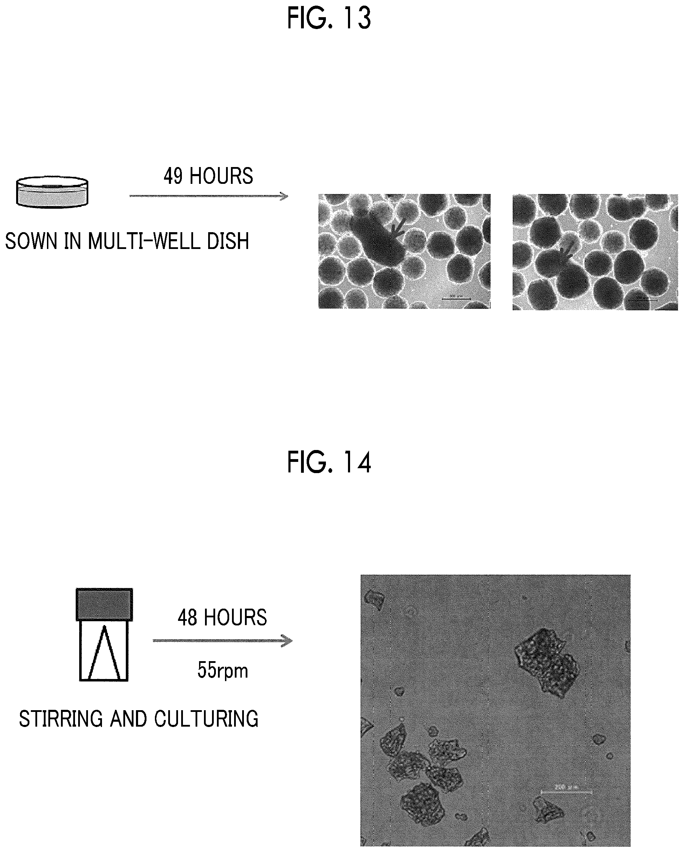

The above 90 mm dish was allowed to stand in a $CO_2$ incubator at 37°C for 49 hours, the fusion of the cell structures was recognized, and it was not able to obtain a large number of cell structures having uniform shapes and sizes. (Fig. 13)

[Comparative Example 3] Manufacturing large number of cell structures by stirring and culturing only

Human bone marrow-derived mesenchymal stem cells (hMSCs) were suspended in a growth medium (Takara Bio Inc.: MSCGM BulletKit (Trade Mark)), biocompatible macromolecular blocks (53 - 106 μm) manufactured in Reference Example 3 was added thereto, hMSC ($1.2 \times 10^8$ cells) and biocompatible macromolecular blocks (25 mg) in a state of being suspended in 30 mL of a medium were introduced into a product name of 30 mL single use bioreactor (BWV-S03A manufactured by ABLE Corporation) (culture container comprising stirring means), and stirring and culturing were performed at a rotational speed of 55 rpm for 24 hours or 48 hours. As a result, only several tens of nonuniform cell structures (for example, cellular structures having nonuniform shapes and sizes or cell masses only including cells without including biocompatible macromolecular blocks) were present, and it was not possible to obtain a large number of uniform cell structures as in Examples 1 and 2 (Fig. 14). Fig. 14 illustrates the results of the stirring and culturing for 48 hours.

[Test Example 1] Uniformity evaluation of cell structure

With respect to the cell structures obtained in Examples 1 and 2 and Comparative Examples 1, 2, and 3, the uniformity of the cell structures was evaluated using indexes of fusion avoidance and size uniformity.

With respect to the fusion avoidance, a case where the ratio of the number of manufactured cell structures to the number of multi-wells was 70% or less was B, and a case where the ratio was 70% to 100% was A. That is, with respect to the fusion avoidance, A is more excellent than B. The evaluation results are provided in Table 1.

With respect to the size uniformity, the sizes of the obtained cell structures were evaluated. A case where 70% or more are in the size (the same size means that a case where the size is in ±50% of the median size) is set as A, a case where 40% or more and less than 70% are in the size is set as B, and a case where less than 40% are in the size is set as C. That is, the size uniformity is excellent in A, B is inferior to A, and C is further inferior to B. The evaluation results are provided in Table 1.

[Table 1]

|  | Fusion avoidance | Size uniformity |
|---|---|---|
| Example 1 | A | A |
| Example 2 | A | A |
| Comparative Example 1 | A | B |
| Comparative Example 2 | B | B |
| Comparative Example 3 | Not measurable | C |

In Comparative Example 3, a multi-well was not used, and the fusion avoidance was not able to be measured in terms of an evaluation index, and thus the evaluation was made only based on the size uniformity.

As presented in Table 1, the manufacturing methods of Examples 1 and 2 were excellent in fusion avoidance and size uniformity.

Explanation of References

    10: container main body
    12: lid
    14: bottom plate portion
    16: side wall part
    20: recessed portion
    24: well forming area
    26: culture surface
    30: cell adhesion suppressant layer
    A: irradiation spot
    B: irradiation spot
    C: irradiation spot
    D: irradiation spot

[SEQUENCE LISTING]

International Application 17F02719 Method of manufacturing cell structure JP18010535 20180316----00150237551800558715 Normal 20180316154500201802231506204110_P1AP101_17_1.app Based on International Patent Cooperation Treaty

**Claims**

1. A method of manufacturing a cell structure, comprising:

   a step (A) of adding a mixture of biocompatible macromolecular blocks, cells, and a liquid medium to a first culture container having a culture surface on which a plurality of recessed portions are formed and a side wall part standing on an outer periphery of the culture surface such that a liquid surface of the mixture is over the culture surface;
   a step (B) of allowing a first culture container of the step (A) to stand and forming a cell structure which includes the biocompatible macromolecular block and the cell in the recessed portion and in which the plurality of biocompatible macromolecular blocks are disposed in gaps between the plurality of cells; and
   a step (C) of stirring and culturing a content of the first culture container obtained in the step (B) in a second culture container comprising stirring means,

   (i) wherein the first culture container is allowed to stand in the step (B) for a period of time for which a ratio of the number of cell structures manufactured after the step (C) to the number of recessed portions is 70% or more,
   (ii) wherein the recessed portions are formed by 10 recessed portions/cm$^2$ to 10,000 recessed portions/cm$^2$ per unit area of the well forming area (24), and (iii) wherein the cells to be used are one or more selected from the group consisting of ES cells, iPS cells, MSCs, chondrocytes, osteoblasts, osteoprecursor cells, mesenchymal cells, myoblasts, cardiac muscle cells, cardiomyoblasts, nerve cells, hepatocytes, beta cells, fibroblasts, corneal endothelial cells, vascular endothelial cells, corneal epithelial cells, amniotic cells, umbilical cord blood cells, bone marrow derived cells, and hematopoietic stem cells.

2. The method of manufacturing a cell structure according to claim 1,
   wherein the first culture container is allowed to stand in the step (B) until free biocompatible macromolecular blocks are 30 mass% or less of the total biocompatible macromolecular blocks.

3. The method of manufacturing a cell structure according to any one of claims 1 or 2,
   wherein a size of the biocompatible macromolecular block is 1 $\mu$m to 700 $\mu$m.

4. The method of manufacturing a cell structure according to any one of claims 1 to 3,
   wherein a treatment for suppressing cell adhesion is performed on the culture surface and a surface of the recessed portion.

5. The method of manufacturing a cell structure according to any one of claims 1 to 4,
   wherein a depth of the recessed portion is 2 to 100 times of a size of the biocompatible macromolecular block, and a diameter of the recessed portion is 2 to 100 times of a size of the biocompatible macromolecular block.

6. The method of manufacturing a cell structure according to any one of claims 1 to 5,
   wherein a period of time for which the first culture container is allowed to stand is 2 hours to 24 hours.

7. The method of manufacturing a cell structure according to any one of claims 1 to 6,
   wherein stirring and culturing time for which a content of the first culture container in the step (C) is 12 hours to 93 hours.

8. The method of manufacturing a cell structure according to any one of claims 1 to 7,
   wherein the biocompatible macromolecule is gelatin.

9. The method of manufacturing a cell structure according to claim 8,

   wherein the gelatin is represented by the following formula.

   Formula:          A-[(Gly-X-Y)$_n$]$_m$-B

in the formula, A represents random amino acid or an amino acid sequence, B represents any amino acid or amino acid sequence, n X's each independently represent any one of amino acid, n Y's each independently represent any one of any amino acid, n represents an integer of 3 to 100, and m represents an integer of 2 to 10, and n Gly-X-Y's may be identical to or different from each other.

10. The method of manufacturing a cell structure according to claim 8 or 9,

wherein the gelatin is any one of
a peptide including an amino acid sequence described in SEQ ID NO: 1;
a peptide having biocompatibility and including an amino acid sequence in which one or more amino acids are deleted, substituted, or added in an amino acid sequence described in

SEQ ID NO: 1; or
a peptide which is formed of an amino acid sequence having 80% or more sequence identity to the amino acid sequence described in SEQ ID No: 1, and has biocompatibility.


**Patentansprüche**

1. Verfahren zum Herstellen einer Zellstruktur, umfassend:

einen Schritt (A) des Hinzufügens einer Mischung von biokompatiblen makromolekularen Blöcken, Zellen und einem flüssigen Medium zu einem ersten Kulturcontainer mit einer Kulturoberfläche, auf der eine Vielzahl von vertieften Teilen gebildet sind, und einem Seitenwandteil, der auf einer äußeren Peripherie der Kulturoberfläche steht, so dass eine Flüssigkeitsoberfläche der Mischung über der Kulturoberfläche liegt;
einen Schritt (B) des Stehenlassens eines ersten Kulturcontainers von Schritt (A) und Bildung einer Zellstruktur, die die biokompatiblen makromolekularen Blöcke und die Zelle in dem vertieften Teil einschließt, und in welcher die Vielzahl von biokompatiblen makromolekularen Blöcken in Lücken zwischen der Vielzahl von Zellen angeordnet sind; und
einen Schritt (C) des Rührens und Kultivierens eines in Schritt (B) erhaltenen Inhalts des ersten Kulturcontainers in einem zweiten Kulturcontainer, der Mittel zum Rühren umfasst,

(i) wobei der erste Kulturcontainer in Schritt (B) für einen Zeitraum stehen gelassen wird, für welchen ein Verhältnis der Anzahl der nach Schritt (C) hergestellten Zellstrukturen zu der Anzahl von vertiefen Teilen 70% oder mehr beträgt,
(ii) wobei die vertieften Abschnitte durch 10 vertiefte Abschnitte/cm$^2$ bis 10.000 vertiefte Abschnitte/cm$^2$ pro Flächeneinheit der Well-bildenden Fläche (24) gebildet werden, und
(iii) wobei die zu verwendenden Zellen eines oder mehrere sind ausgewählt aus der Gruppe bestehend aus ES-Zellen, iPS-Zellen, MSCs, Chondrozyten, Osteoblasten, Osteovorläuferzellen, mesenchymalen Zellen, Myoblasten, Herzmuskelzellen, Kardiomyoblasten, Nervenzellen, Hepatozyten, Beta-Zellen, Fibroblasten, kornealen Endothelzellen, vaskulären Endothelzellen, kornealen Epithelzellen, Amnionzellen, Nabelschnurblutzellen, vom Knochenmark abgeleiteten Zellen und hämatopoetischen Stammzellen.

2. Verfahren zum Herstellen einer Zellstruktur gemäß Anspruch 1,
wobei der erste Kulturcontainer in Schritt (B) so lange stehen gelassen wird, bis freie biokompatible makromolekulare Blöcke 30 Massen-% oder weniger der gesamten biokompatiblen makromolekularen Blöcke ausmachen.

3. Verfahren zum Herstellen einer Zellstruktur gemäß irgendeinem der Ansprüche 1 oder 2,
wobei eine Größe des biokompatiblen makromolekularen Blocks 1 μm bis 700 μm beträgt.

4. Verfahren zum Herstellen einer Zellstruktur gemäß irgendeinem der Ansprüche 1 bis 3,
wobei eine Behandlung zum Unterdrücken von Zelladhäsion auf der Kulturoberfläche und einer Oberfläche des vertieften Teils durchgeführt wird.

5. Verfahren zum Herstellen einer Zellstruktur gemäß irgendeinem der Ansprüche 1 bis 4,
wobei eine Tiefe des vertieften Teils 2 bis 100-mal von der Größe des biokompatiblen makromolekularen Blocks beträgt, und ein Durchmesser des vertieften Teils 2 bis 100-mal von der Größe des biokompatiblen makromolekularen Blocks beträgt.

**6.** Verfahren zum Herstellen einer Zellstruktur gemäß irgendeinem der Ansprüche 1 bis 5, wobei ein Zeitraum, für welchen der erste Kulturcontainer stehen gelassen wird 2 Stunden bis 24 Stunden beträgt.

**7.** Verfahren zum Herstellen einer Zellstruktur gemäß irgendeinem der Ansprüche 1 bis 6, wobei Rühr- und Kultivierungszeit, für welchen ein Inhalt des ersten Kulturcontainers in dem Schritt (C) 12 Stunden bis 93 Stunden beträgt.

**8.** Verfahren zum Herstellen einer Zellstruktur gemäß irgendeinem der Ansprüche 1 bis 7, wobei das biokompatible Makromolekül Gelatine ist.

**9.** Verfahren zum Herstellen einer Zellstruktur gemäß Anspruch 8,

wobei die Gelatine durch die folgende Formel dargestellt wird

$$\text{Formel:} \qquad A\text{-}[(Gly\text{-}X\text{-}Y)_n)]_m\text{-}B$$

In der Formel repräsentiert A willkürliche Aminosäure oder eine Aminosäuresequenz, repräsentiert B irgendeine Aminosäure oder Aminosäuresequenz, n X repräsentieren jeweils unabhängig voneinander irgend eine von Aminosäure, n Y repräsentieren jeweils unabhängig voneinander irgend eine von irgendeiner Aminosäure, n repräsentiert eine ganze Zahl von 3 bis 100 und m repräsentiert eine ganze Zahl von 2 bis 10 und n Gly-X-Y können identisch zueinander oder unterschiedlich voneinander sein.

**10.** Verfahren zum Herstellen einer Zellstruktur gemäß Anspruch 8 oder 9,

wobei die Gelatine irgendeins ist von ein Peptid, das eine in SEQ ID NO: 1 beschriebene Aminosäuresequenz einschließt;
ein Peptid mit Biokompatibilität und einschließend eine Aminosäuresequenz, in welcher eine oder mehrere Aminosäuren in einer in SEQ ID NO: 1 beschriebenen Aminosäuresequenz gelöscht, substituiert oder addiert sind; oder
ein Peptid, welches aus eine Aminosäuresequenz mit 80% oder mehr Sequenzidentität zu der in SEQ ID NO: 1 beschriebenen Aminosäuresequenz gebildet wird und Biokompatibilität aufweist.

**Revendications**

**1.** Procédé de fabrication d'une structure cellulaire, comprenant :

l'étape (A) consistant à ajouter un mélange de séquences macromoléculaires biocompatibles, de cellules et d'un milieu liquide à un premier récipient de culture présentant une surface de culture sur laquelle une pluralité de portions évidées sont formées et une partie de paroi latérale reposant sur une périphérie externe de la surface de culture de telle manière qu'une surface liquide du mélange est sur la surface de culture ;
une étape (B) consistant à laisser un premier récipient de culture de l'étape (A) reposer et former une structure cellulaire qui inclut la séquence macromoléculaire biocompatible et la cellule dans la portion évidée et dans laquelle la pluralité de séquences macromoléculaires biocompatibles sont disposées dans des espaces entre la pluralité de cellules ; et
l'étape (C) consistant à agiter et cultiver un contenu du premier récipient de culture obtenu dans l'étape (B) dans un second récipient de culture comprenant un moyen d'agitation,

(i) dans lequel le premier récipient de culture est laissé à reposer dans l'étape (B) pendant une période de temps pendant laquelle un rapport du nombre de structures cellulaires fabriquées après l'étape (C) au nombre de portions évidées est de 70 % ou plus,
(ii) dans lequel les portions évidées sont formées par 10 portions évidées/cm$^2$ à 10 000 portions évidées/cm$^2$ par unité de surface de la zone formant des puits (24), et (iii) dans lequel les cellules devant être utilisées sont une ou plusieurs choisies dans le groupe constitué de cellules souches embryonnaires, de cellules souches pluripotentes induites, de cellules souches mésenchymateuses, de chondrocytes, d'ostéoblastes, de cellules ostéoprécurseurs, de cellules mésenchymateuses, de myoblastes, de cellules de muscle cardiaque, de cardiomyoblastes, de cellules nerveuses, d'hépatocytes, de cellules bêta, de fibroblastes, de cellules endothéliales cornéennes, de cellules endothéliales vasculaires, de cellules épithéliales cornéennes, de cellules amniotiques, de globules de cordon ombilical, de cellules dérivées de la moelle osseuse et de cellules souches hématopoïétiques.

2. Procédé de fabrication d'une structure cellulaire selon la revendication 1, dans lequel le premier récipient de culture est laissé à reposer dans l'étape (B) jusqu'à ce que des séquences macromoléculaires biocompatibles libres représentent 30 % en masse ou moins des séquences macromoléculaires biocompatibles totales.

3. Procédé de fabrication d'une structure cellulaire selon l'une quelconque des revendications 1 ou 2, dans lequel une taille de la séquence macromoléculaire biocompatible est de 1 $\mu$m à 700 $\mu$m.

4. Procédé de fabrication d'une structure cellulaire selon l'une quelconque des revendications 1 à 3, dans lequel un traitement pour supprimer l'adhérence cellulaire est réalisé sur la surface de culture et une surface de la portion évidée.

5. Procédé de fabrication d'une structure cellulaire selon l'une quelconque des revendications 1 à 4, dans lequel une profondeur de la portion évidée est 2 à 100 fois une taille de la séquence macromoléculaire biocompatible, et un diamètre de la portion évidée est 2 à 100 fois une taille de la séquence macromoléculaire biocompatible.

6. Procédé de fabrication d'une structure cellulaire selon l'une quelconque des revendications 1 à 5, dans lequel une période de temps pendant laquelle le premier récipient de culture est laissé à reposer est de 2 heures à 24 heures.

7. Procédé de fabrication d'une structure cellulaire selon l'une quelconque des revendications 1 à 6, dans lequel le temps d'agitation et de culture pour un contenu du premier récipient de culture dans l'étape (C) est de 12 heures à 93 heures.

8. Procédé de fabrication d'une structure cellulaire selon l'une quelconque des revendications 1 à 7, dans lequel la macromolécule biocompatible est de la gélatine.

9. Procédé de fabrication d'une structure cellulaire selon la revendication 8,

dans lequel la gélatine est représentée par la formule suivante.

Formule :     A-[(Gly-X-Y)$_n$]$_m$-B

dans la formule, A représente un acide aminé aléatoire ou une séquence d'acides aminés, B représente un acide aminé quelconque ou une séquence d'acides aminés, n X représentent chacun indépendamment un acide aminé quelconque, n Y représentent chacun indépendamment un acide aminé quelconque, n représente un nombre entier de 3 à 100, et m représente un nombre entier de 2 à 10, et n Gly-X-Y peuvent être identiques les uns aux autres ou différents les uns des autres.

10. Procédé de fabrication d'une structure cellulaire selon la revendication 8 ou 9, dans lequel la gélatine est l'un quelconque de

un peptide incluant une séquence d'acides aminés décrite dans la SEQ ID N° : 1 ;
un peptide présentant une biocompatibilité et incluant une séquence d'acides aminés dans laquelle un ou plusieurs acides aminés sont supprimés, substitués ou ajoutés dans une séquence d'acides aminés décrite dans la SEQ ID N° : 1 ; ou
un peptide qui est formé d'une séquence d'acides aminés présentant 80 % ou plus d'identité de séquence avec la séquence d'acides aminés décrite dans la SEQ ID N° : 1, et présente une biocompatibilité.

## FIG. 2A

## FIG. 2B

## FIG. 3A

## FIG. 3B

FIG. 4

DIAMETER

DEPTH

26

FIG. 5

DIAMETER

DEPTH

26

# FIG. 6

## FIG. 7

EP 3 597 731 B1

FIG. 8

EP 3 597 731 B1

## FIG. 9

FIG. 10

FIG. 11

FIG. 12

SOWN IN MULTI-WELL DISH

FIVE HOURS

SIZE OF CELL STRUCTURE

# FIG. 13

49 HOURS

SOWN IN MULTI-WELL DISH

# FIG. 14

48 HOURS

55rpm

STIRRING AND CULTURING

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 3156081 A
- EP 3358003 A
- EP 3473259 A
- WO 2011108517 A
- JP 2014012114 A
- WO 2014133081 A
- WO 2012036011 A

- JP 2015073520 A
- EP 1014176 A
- US 6992172 B
- WO 2004085473 A
- WO 2008103041 A
- EP 1014176 A2


**Non-patent literature cited in the description**

- *Pharmaceutical Bulletin,* 1954, vol. 2 (2), 163-173
- *Area of Chemistry,* 1957, vol. 11 (10), 719-725
- *Fragrance Journal,* 1981, vol. 50, 79-82
- **YOSHIO KOUDA.** New Edition Organic Conceptual Diagram -Foundation and Application. Sankyo Shuppan Co., Ltd, 2008
- Protein Identification and Analysis Tools on the ExPASy Server. **GASTEIGER E. ; HOOGLAND C. ; GATTIKER A. ; DUVAUD S. ; WILKINS M.R. ; APPEL R.D. ; BAIROCH A.** The Proteomics Protocols Handbook. Humana Press, 2005, 571-607

- ExPASy: the proteomics server for in-depth protein knowledge and analysis. **GASTEIGER E. ; GATTIKER A. ; HOOGLAND C. ; IVANYI I. ; APPEAL R.D. ; BAIROCH A.** Nucleic Acids Res. 2003, vol. 31, 3784-3788
- Pathophysiology. Nagai Shoten Co., Ltd, 1990, vol. 9, 527
- **MAXEY, C. R.** Phitogr. Gelatin. P. J. Academic, 1976, vol. 2

SEQUENCE LISTING

<110> FUJIFILM Corporation
<120> A CELL CONSTRUCT AND A METHOD FOR PRODUCING CELL CONSTRUCT
<130>\201@17F02719
<160> 10
<170> PatentIn version 3.5
<210> 1
<211> 571
<212> PRT
<213> Recombinant
<400> 1

```
Gly Ala Pro Gly Ala Pro Gly Leu Gln Gly Ala Pro Gly Leu Gln Gly
1               5                   10                  15
Met Pro Gly Glu Arg Gly Ala Ala Gly Leu Pro Gly Pro Lys Gly Glu
                20                  25                  30
Arg Gly Asp Ala Gly Pro Lys Gly Ala Asp Gly Ala Pro Gly Ala Pro
            35                  40                  45
Gly Leu Gln Gly Met Pro Gly Glu Arg Gly Ala Ala Gly Leu Pro Gly
        50                  55                  60
Pro Lys Gly Glu Arg Gly Asp Ala Gly Pro Lys Gly Ala Asp Gly Ala
65                  70                  75                  80
Pro Gly Lys Asp Gly Val Arg Gly Leu Ala Gly Pro Ile Gly Pro Pro
                85                  90                  95
Gly Glu Arg Gly Ala Ala Gly Leu Pro Gly Pro Lys Gly Glu Arg Gly
            100                 105                 110
Asp Ala Gly Pro Lys Gly Ala Asp Gly Ala Pro Gly Lys Asp Gly Val
        115                 120                 125
Arg Gly Leu Ala Gly Pro Ile Gly Pro Pro Gly Pro Ala Gly Ala Pro
    130                 135                 140
Gly Ala Pro Gly Leu Gln Gly Met Pro Gly Glu Arg Gly Ala Ala Gly
145                 150                 155                 160
Leu Pro Gly Pro Lys Gly Glu Arg Gly Asp Ala Gly Pro Lys Gly Ala
            165                 170                 175
Asp Gly Ala Pro Gly Lys Asp Gly Val Arg Gly Leu Ala Gly Pro Pro
        180                 185                 190
Gly Ala Pro Gly Leu Gln Gly Ala Pro Gly Leu Gln Gly Met Pro Gly
        195                 200                 205
Glu Arg Gly Ala Ala Gly Leu Pro Gly Pro Lys Gly Glu Arg Gly Asp
    210                 215                 220
Ala Gly Pro Lys Gly Ala Asp Gly Ala Pro Gly Ala Pro Gly Leu Gln
225                 230                 235                 240
Gly Met Pro Gly Glu Arg Gly Ala Ala Gly Leu Pro Gly Pro Lys Gly
            245                 250                 255
Glu Arg Gly Asp Ala Gly Pro Lys Gly Ala Asp Gly Ala Pro Gly Lys
            260                 265                 270
Asp Gly Val Arg Gly Leu Ala Gly Pro Ile Gly Pro Pro Gly Glu Arg
        275                 280                 285
Gly Ala Ala Gly Leu Pro Gly Pro Lys Gly Glu Arg Gly Asp Ala Gly
    290                 295                 300
Pro Lys Gly Ala Asp Gly Ala Pro Gly Lys Asp Gly Val Arg Gly Leu
305                 310                 315                 320
Ala Gly Pro Ile Gly Pro Pro Gly Pro Ala Gly Ala Pro Gly Ala Pro
            325                 330                 335
Gly Leu Gln Gly Met Pro Gly Glu Arg Gly Ala Ala Gly Leu Pro Gly
        340                 345                 350
Pro Lys Gly Glu Arg Gly Asp Ala Gly Pro Lys Gly Ala Asp Gly Ala
        355                 360                 365
Pro Gly Lys Asp Gly Val Arg Gly Leu Ala Gly Pro Pro Gly Ala Pro
    370                 375                 380
Gly Leu Gln Gly Ala Pro Gly Leu Gln Gly Met Pro Gly Glu Arg Gly
385                 390                 395                 400
Ala Ala Gly Leu Pro Gly Pro Lys Gly Glu Arg Gly Asp Ala Gly Pro
            405                 410                 415
```

24

```
Lys Gly Ala Asp Gly Ala Pro Gly Ala Pro Gly Leu Gln Gly Met Pro
            420                 425                 430
Gly Glu Arg Gly Ala Ala Gly Leu Pro Gly Pro Lys Gly Glu Arg Gly
            435                 440                 445
Asp Ala Gly Pro Lys Gly Ala Asp Gly Ala Pro Gly Lys Asp Gly Val
            450                 455                 460
Arg Gly Leu Ala Gly Pro Ile Gly Pro Pro Gly Glu Arg Gly Ala Ala
465                 470                 475                 480
Gly Leu Pro Gly Pro Lys Gly Glu Arg Gly Asp Ala Gly Pro Lys Gly
                485                 490                 495
Ala Asp Gly Ala Pro Gly Lys Asp Gly Val Arg Gly Leu Ala Gly Pro
                500                 505                 510
Ile Gly Pro Pro Gly Pro Ala Gly Ala Pro Gly Ala Pro Gly Leu Gln
            515                 520                 525
Gly Met Pro Gly Glu Arg Gly Ala Ala Gly Leu Pro Gly Pro Lys Gly
            530                 535                 540
Glu Arg Gly Asp Ala Gly Pro Lys Gly Ala Asp Gly Ala Pro Gly Lys
545                 550                 555                 560
Asp Gly Val Arg Gly Leu Ala Gly Pro Pro Gly
                565                 570
```

<210> 2
<211> 4
<212> PRT
<213> Artificial Sequence
<220>
<223> Description of Artificial Sequence: adhesive sequence
<400> 2

```
                        Arg Glu Asp Val
                        1
```

<210> 3
<211> 5
<212> PRT
<213> Artificial Sequence
<220>
<223> Description of Artificial Sequence: adhesive sequence
<400> 3

```
                        Tyr Ile Gly Ser Arg
                        1               5
```

<210> 4
<211> 5
<212> PRT
<213> Artificial Sequence
<220>
<223> Description of Artificial Sequence: adhesive sequence
<400> 4

```
                        Pro Asp Ser Gly Arg
                        1               5
```

<210> 5
<211> 7
<212> PRT
<213> Artificial Sequence
<220>

25

<223> Description of Artificial Sequence: adhesive sequence
<400> 5

```
                        Arg Tyr Val Val Leu Pro Arg
                        1               5
```

<210> 6
<211> 6
<212> PRT
<213> Artificial Sequence
<220>
<223> Description of Artificial Sequence: adhesive sequence
<400> 6

```
                        Leu Gly Thr Ile Pro Gly
                        1               5
```

<210> 7
<211> 10
<212> PRT
<213> Artificial Sequence
<220>
<223> Description of Artificial Sequence: adhesive sequence
<400> 7

```
                  Arg Asn Ile Ala Glu Ile Ile Lys Asp Ile
                  1               5                   10
```

<210> 8
<211> 5
<212> PRT
<213> Artificial Sequence
<220>
<223> Description of Artificial Sequence: adhesive sequence
<400> 8

```
                        Ile Lys Val Ala Val
                        1               5
```

<210> 9
<211> 4
<212> PRT
<213> Artificial Sequence
<220>
<223> Description of Artificial Sequence: adhesive sequence
<400> 9

```
                        Asp Gly Glu Ala
                        1
```

<210> 10
<211> 4
<212> PRT
<213> Artificial Sequence
<220>
<223> Description of Artificial Sequence: adhesive sequence
<400> 10

```
Glu Arg Gly Asp
1
```